(19) Europäisches Patentamt
European Patent Office
Office européen des brevets

(11) Veröffentlichungsnummer: **0 075 146**
**B1**

(12) # EUROPÄISCHE PATENTSCHRIFT

(45) Veröffentlichungstag der Patentschrift: **23.10.85**

(21) Anmeldenummer: **82108000.9**

(22) Anmeldetag: **31.08.82**

(51) Int. Cl.⁴: **C 07 C 69/743,** C 07 C 121/75, C 07 D 317/46, C 07 C 67/08, C 07 C 43/23, C 07 C 29/14, C 07 C 41/26, C 07 C 47/575, C 07 C 45/41, C 07 C 45/42, A 01 N 53/00

(54) **Benzylester mit fluorsubstituierten Ethergruppen, Verfahren zu ihrer Herstellung und ihre Verwendung als Schädlingsbekämpfungsmittel.**

(30) Priorität: **10.09.81 DE 3135926**

(43) Veröffentlichungstag der Anmeldung: **30.03.83 Patentblatt 83/13**

(45) Bekanntmachung des Hinweises auf die Patenterteilung: **23.10.85 Patentblatt 85/43**

(84) Benannte Vertragsstaaten: **BE CH DE FR GB IT LI NL**

(56) Entgegenhaltungen:
**EP - A - 0 006 978**
**EP - A - 0 041 131**
**DE - A - 2 333 849**
**GB - A - 2 046 732**

**Die Akte enthält technische Angaben, die nach dem Eingang der Anmeldung eingereicht wurden und die nicht in dieser Patentschrift enthalten sind.**

(73) Patentinhaber: **BAYER AG, Konzernverwaltung RP Patentabteilung, D-5090 Leverkusen 1 Bayerwerk (DE)**

(72) Erfinder: **Lantzsch, Reinhard, Dr., Heymannstrasse 32, D-5090 Leverkusen 1 (DE)**
Erfinder: **Marhold, Albrecht, Dr., Carl-Duisberg-Strasse 329, D-5090 Leverkusen 1 (DE)**
Erfinder: **Behrenz, Wolfgang, Dr., Untergründemich 14, D-5063 Overath (DE)**
Erfinder: **Hammann, Ingeborg, Dr., Belfortstrasse 9, D-5000 Köln 1 (DE)**
Erfinder: **Homeyer, Bernhard, Dr., Obere Strasse 28, D-5090 Leverkusen 3 (DE)**
Erfinder: **Stendel, Wilhelm, Dr., In den Birken 55, D-5600 Wuppertal 1 (DE)**

EP 0 075 146 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft neue Benzylester mit fluorsubstituierten Ethergruppen, Verfahren zu ihrer Herstellung, ihre Verwendung als Schädlingsbekämpfungsmittel, sowie neue Zwischenprodukte zur Herstellung dieser Wirkstoffe.

Es ist bereits bekannt geworden, daß 3-Phenoxy substituierte Benzylester von Vinylcyclopropancarbonsäuren insektizid wirksam sind; so z. B. 2,2-Dimethyl-3-(β,β-dimethylvinyl)-cyclopropancarbonsäure-(3-phenoxy)-benzylester.

Die Wirkung dieser Verbindungen ist jedoch bei niedrigen Aufwandkonzentrationen nicht immer voll befriedigend.

Weiterhin sind aus EP-A 6 978 ähnlich strukturierte Benzylester bekannt, wie z. B. 3-Trifluormethoxy-2,4,5,6-tetrachlor-benzyl-2,2-dimethyl-3-(2,2-dichlorvinyl)-cyclopropan-carboxylat oder 3-Trifluormethoxy-benzyl-2,2-dimethyl-3-(2,2-dichlorvinyl)-cyclopropancarboxylat.

Gegenüber diesen eng verwandten Verbindungen zeigen die erfindungsgemäßen Wirkstoffe jedoch im überraschenden Maße überlegene biologische Eigenschaften.

1. Es wurden Benzylester der Formel (I) gefunden,

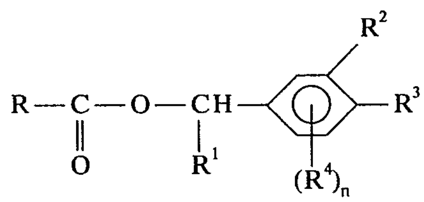

(I)

in welcher

R für den Rest einer bei Pyrethroiden verwendbaren Säure steht,
$R^1$ für Wasserstoff oder Cyan steht,
$R^2$ für Trifluormethoxy steht und
$R^3$ für Wasserstoff oder Halogen steht oder
$R^2$ und $R^3$ gemeinsam für fluorsubstituiertes Dioxymethylen stehen,
$R^4$ für Halogen steht und
n für 0, 1, 2 oder 3 steht,

mit der Maßgabe, daß für den Fall, daß $R^2$ für $OCF_3$ steht, einer der Reste $R^3$ oder $R^4$ für Fluor stehen muß und ferner mit der Maßgabe, daß wenn $R^2$ und $R^3$ für fluorsubstituiertes Dioxymethylen stehen, n für 1 steht und $R^4$ in 6-Stellung für Fluor stehen muß.

2. Es wurde ferner gefunden, daß man die neuen Benzylester der Formel (I)

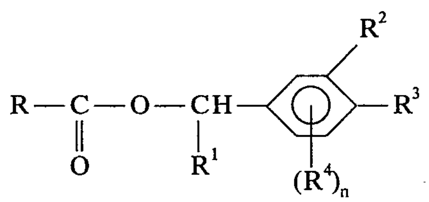

(I)

in welcher

R für den Rest einer bei Pyrethroiden verwendbaren Säure steht,
$R^1$ für Wasserstoff oder Cyan steht,
$R^2$ für Trifluormethoxy steht und
$R^3$ für Wasserstoff oder Halogen steht oder
$R^2$ und $R^3$ gemeinsam für fluorsubstituiertes Dioxymethylen stehen,
$R^4$ für Halogen steht und
n für 0, 1, 2 oder 3 steht,

mit der Maßgabe, daß für den Fall, daß $R^2$ für $OCF_3$ steht, einer der Reste $R^3$ oder $R^4$ für Fluor stehen muß und ferner mit der Maßgabe, daß wenn $R^2$ und $R^3$ für fluorsubstituiertes Dioxymethylen stehen, n für 1 steht und $R^4$ in 6-Stellung für Fluor stehen muß, erhält, indem man eine bei Pyrethroiden verwendbare Säure oder ein reaktionsfähiges Derivat derselben der Formel (II)

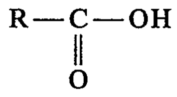 (II)

in welcher
R die oben angegebene Bedeutung besitzt,
mit einem Alkohol oder einem reaktionsfähigen Derivat desselben der Formel (III)

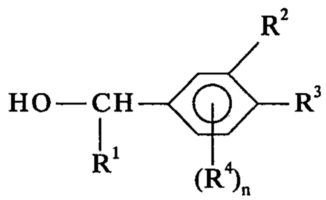 (III)

in welcher
$R^1$–$R^4$ und n die oben angegebene Bedeutung besitzen,
umgesetzt.

3. Es wurden ferner die neuen Benzylalkohle der Formel (III)

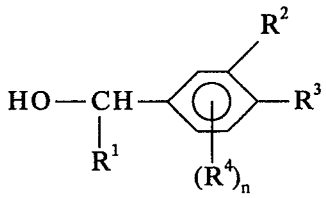 (III)

in welcher

$R^1$ für Wasserstoff oder Cyan steht,
$R^2$ für Trifluormethoxy steht und
$R^3$ für Wasserstoff oder Halogen steht oder
$R^2$ und $R^3$ gemeinsam für fluorsubstituiertes Dioxymethylen stehen,
$R^4$ für Halogen steht und
n für 0, 1, 2 oder 3 steht,

mit der Maßgabe, daß für den Fall, daß $R^2$ für $OCF_3$ steht, einer der Reste $R^3$ oder $R^4$ für Fluor stehen muß und ferner mit der Maßgabe, daß wenn $R^2$ und $R^3$ für fluorsubstituiertes Dioxymethylen stehen, n für 1 steht und $R^4$ in 6-Stellung für Fluor stehen muß.

4. Es wurde ferner gefunden, daß man die neuen Benzylalkohole der Formel (III) gemäß 3. (oben) erhält, indem man

a) Aldehyde der Formel (IV)

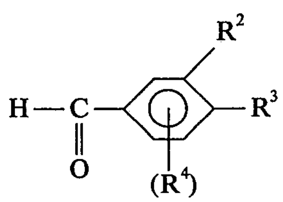 (IV)

in welcher
$R^2$–$R^4$ und n die oben angegebene Bedeutung besitzen

$a_1$) für den Fall, daß $R^1$ für Wasserstoff steht, reduziert oder
$a_2$) für den Fall, daß $R^1$ für Cyan steht, mit Cyaniden (gegebenenfalls in situ) zum Cyanhydrin umsetzt, oder

b) indem man Benzylhalogenide der Formel (V)

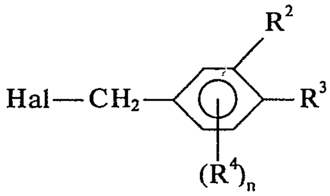

(V)

in welcher $R^2$–$R^4$ und n die oben angegeben Bedeutung haben und Hal für Chlor oder Brom steht, hydrolysiert oder

c) Säurehalogenie der Formel (VI)

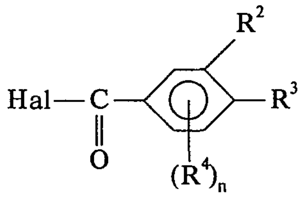

(VI)

in welcher $R^2$–$R^4$ und n die oben angegebene Bedeutung besitzen und Hal für Fluor, Chlor oder Brom steht, mit einem Alkali-Boranat reduziert.

5. Es wurden ferner die neuen Aldehyde der Formel (IV)

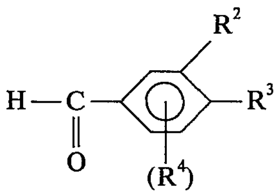

(IV)

in welcher $R^2$–$R^4$ und n die oben angegebene Bedeutung besitzen, gefunden.

6. Es wurde ferner gefunden, daß man die neuen Aldehyde der Formel (IV)

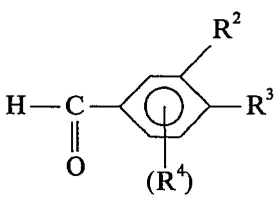

(IV)

in welcher $R^2$–$R^4$ und n die oben angegebene Bedeutung besitzen, dadurch erhält, indem man

a) Benzylhalogenide der Formel (V) mit Urotropin umsetzt und anschließend hydrolysiert oder

b) Verbindungen der Formel (VI)

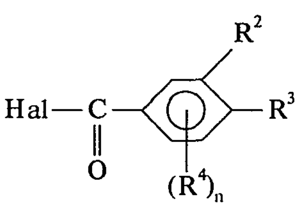

(VI)

in welcher
$R^2$—$R^4$ und n die oben angegebene Bedeutung besitzen und
Hal für Chlor oder Brom steht,
mit Trialkylphosphit umsetzt, anschließend mit Alkaliboranat reduziert und dann hydrolysiert.

7. Es wurden ferner die Benzylhalogenide der Formel (V)

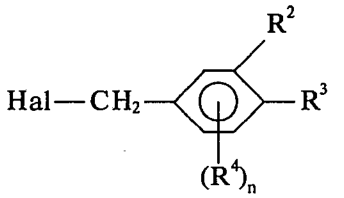

(V)

in welcher
$R^2$—$R^4$ und n die oben angegebene Bedeutung haben und
Hal für Chlor oder Brom steht,
gefunden.

8. Es wurde ferner gefunden, daß man die Benzylhalogenide der Formel (V) gemäß 7. (oben) erhält, indem man Verbindungen der Formel (VII)

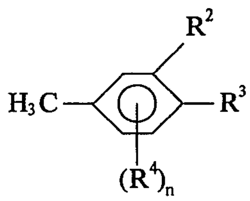

(VII)

in welcher
$R^2$—$R^4$ und n die oben angegebene Bedeutung besitzen,
unter radikalischen Bedingungen halogenisiert.

9. Es wurden ferner die Verbindungen der Formel (VI)

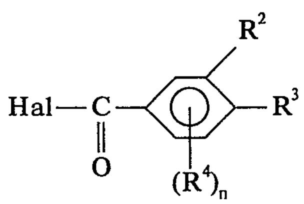

(VI)

in welcher
$R^2$—$R^4$ und n die oben angegebene Bedeutung besitzen und
Hal für Chlor oder Brom steht,
gefunden.

10. Es wurde ferner gefunden, daß man die Verbindungen der Formel (VI) gemäß 9. (oben) erhält indem man Säurehalogenide der Formel (VIII)

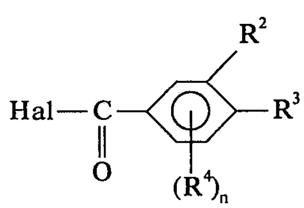

(VIII)

in welcher

Hal für Fluor, Chlor oder Brom steht, und wenn $R^2$ für $OCF_3$ steht,
$R^3$ Chlor oder Brom bedeutet und
$R^4$ Chlor oder Brom bedeutet und
n 0, 1, 2 oder 3 bedeutet

und wenn

$R^2$ und $R^3$ für fluorsubstituiertes Dioxymethylen steht, $R^4$ ein Chlor- oder Bromatom in 6-Stellung bedeutet (n = 1)

mit Kaliumfluorid in einem geeigneten hochsiedenden Lösungsmittel umsetzt und gegebenenfalls das entstandene Säurefluorid in üblicher Weise in ein Säurechlorid oder -bromid überführt.

11. Es wurden ferner die Toluole der Formel (VII)

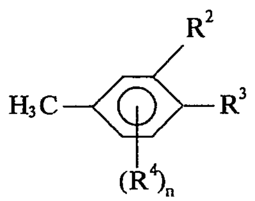

(VII)

in welcher

$R^2$–$R^4$ und n die gemäß 10. (oben) angegebene Bedeutung besitzen,

gefunden.

12. Es wurde ferner gefunden, daß man die Toluole der Formel (VII) gemäß 11 (oben) erhält, indem man

a) Aniline der Formel (IX)

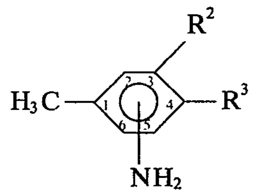

(IX)

in welcher

die Aminogruppen in 5- oder 6-Stellung steht,

$R^2$ für $OCF_3$ steht

$R^3$ für Wasserstoff oder Halogen steht und

$R^2$ und $R^3$ gemeinsam für fluorsubstituiertes Dioxymethylen stehen und die Aminogruppe in 6-Stellung steht,

einer Umsetzung mit Nitrosylfluoroborat unterwirft (Schiemann-Reaktor) oder

b) Phenole der Formel (X)

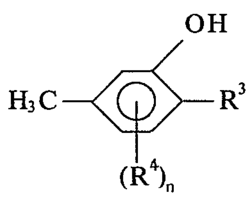

(X)

in welcher

$R^3$ Wasserstoff oder Fluor bedeutet und

$R^4$ für Fluor steht, wobei

n für 0 oder 1 steht, mit der Maßgabe, daß $R^3$ für Fluor steht, wenn n für 0 steht,

mit Fluorwasserstoff in Gegenwart von Tetrachlorkohlenstoff umsetzt.

13. Es wurden ferner die Aniline der Formel (IX)

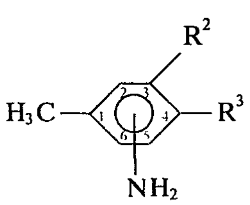

(IX)

in welcher
die Aminogruppe in 5- oder 6-Stellung steht,

$R^2$ für $OCF_3$ steht
$R^3$ für Wasserstoff oder Halogen steht und
$R^2$ und $R^3$ gemeinsam für fluorsubstituiertes Dioxymethylen stehen und die Aminogruppe in 6-Stellung steht,

gefunden.

14. Es wurde ferner gefunden, daß man die Aniline der Formel (IX) gemäß 13 (oben) erhält, indem man

a) für den Fall, daß $R^2$ $OCF_3$ bedeutet, Verbindungen der Formel (XI)

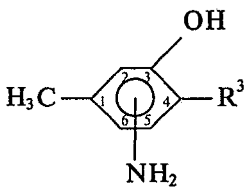

(XI)

in welcher
$R^3$ für Wasserstoff oder Halogen steht und die Aminogruppe in 5- oder 6-Position steht und mit Fluorwasserstoff in Gegenwart von Tetrachlorkohlenstoff umsetzt, oder

b) für den Fall, daß $R^2$ und $R^3$ gemeinsam fluorsubstituiertes Dioxymethylen bedeuten, die Verbindung der Formel (XII)

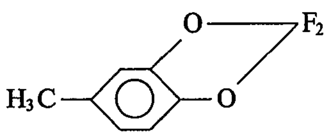

(XII)

nitriert und reduziert wird.

Die Verbindungen der Formel (I) gemäß 1 (oben) zeigen pestizide Eigenschaften. Die allgemeine Formel (I) schließt dabei die verschiedenen möglichen Stereoisomeren, die optischen Isomeren und Mischungen dieser Komponenten ein.

Überraschenderweise zeigen die neuen erfindungsgemäßen Wirkstoffe gemäß 1 (oben) eine erhebliche höhere Wirksamkeit als die aus dem Stand der Technik bekannten Verbindungen.

Von den erfindungsgemäßen Verbindungen der Formel (I) gemäß 1 (oben) sind diejenigen der Formel (XIII) bevorzugt

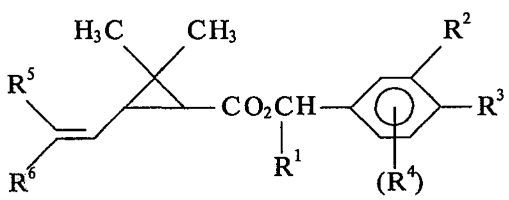

(XIII)

in welcher

$R^1$ für Wasserstoff oder Cyan steht,
$R^2$ für Trifluormethoxy steht,
$R^3$ für Wasserstoff oder Fluor steht oder
$R^2$ und $R^3$ gemeinsam für Difluordioxymethylen stehen,
$R^4$ für Fluor oder Chlor steht und
n für 0, 1, 2, 3 stehen kann, mit der Maßgabe, daß für den Fall, daß $R^2$ für $OCF_3$ steht, einer der Reste $R^3$ oder $R^4$ für Fluor stehen muß und ferner mit der Maßgabe, daß wenn
$R^2$ und $R^3$ für fluorsubstituiertes Dioxymethylen stehen, n für 1 steht und $R^4$ in 6-Stellung für Fluor stehen muß,
$R^5$ und $R^6$ gleich oder verschieden sind und für Methyl, Halogen oder Halogenalkyl mit 1—4 Kohlenstoffatomen stehen.

Besonders bevorzugt sind Verbindungen der Formel (XIII), in welcher

$R^1$–$R^4$ und n die oben als bevorzugt angegebene Bedeutungen besitzen,
$R^5$ für Chlor steht und
$R^6$ für Chlor, Trifluormethyl oder Pentafluorethyl steht.

Folgende Verbindungen der Formel (I) seien im einzelnen genannt:

Difluor-3,4-dioxymethylen-6-fluor-benzyl-2,2-dimethyl-3-(2,2-dichlorvinyl)-cyclopropancarboxylat,
Difluor-3,4-dioxymethylen-6-fluor-benzyl-2,2-dimethyl-3-(3,3,3-trifluor-2-chlor-1-propenyl)-cyclopropancarboxylat,
Difluor-3,4-dioxymethylen-6-fluor-benzyl-2,2-dimethyl-3-(2-chlor-3,3,4,4,4-pentafluor-1-butenyl)-cyclopropancarboxylat,
3-Trifluormethoxy-4-fluor-benzyl-2,2-dimethyl-3-(2,2-dichlorvinyl)-cyclopropancarboxylat,
3-Trifluormethoxy-4-fluor-benzyl-2,2-dimethyl-3-(3,3,3-trifluor-2-chlor-1-propenyl)-cyclopropancarboxylat,
3-Trifluormethoxy-4-fluor-benzyl-2,2-dimethyl-(2-chlor-3,3,4,4,4-pentafluor-1-butenyl)-cyclopropancarboxylat,
3-Trifluormethoxy-2-fluor-benzyl-2,2-dimethyl-3-(2,2-dichlorvinyl)-cyclopropancarboxylat,
3-Trifluormethoxy-5-fluor-benzyl-2,2-dimethyl-3-(2,2-dichlorvinyl)-cyclopropancarboxylat,
3-Trifluormethoxy-6-fluor-benzyl-2,2-dimethyl-3-(2,2-dichlorvinyl)-cyclopropancarboxylat,
3-Trifluormethoxy-2-fluorbenzyl-2,2-dimethyl-3-(2-chlor-3,3,3-trifluor-1-propenyl)-cyclopropancarboxylat,
3-Trifluormethoxy-2-fluor-benzyl-2,2-dimethyl-3-(2-chlor-3,3,4,4,4-pentafluor-1-butenyl)-cyclopropancarboxylat,
3-Trifluormethoxy-5-fluor-benzyl-2,2-dimethyl-3-(2-chlor-3,3,3-trifluor-1-propenyl)-cyclopropancarboxylat,
3-Trifluormethoxy-5-fluor-benzyl-2,2-dimethyl-3-(2-chlor-3,3,4,4,4-pentafluor-1-butenyl)-cyclopropancarboxylat,
3-Trifluormethoxy-6-fluor-benzyl-2,2-dimethyl-3-(2-chlor-3,3,3-trifluor-1-propenyl)-cyclopropancarboxylat,
3-Trifluormethoxy-6-fluor-benzyl-2,2-dimethyl-3-(2-chlor-3,3,4,4,4-pentafluor-1-butenyl)-cyclopropancarboxylat,
3-Trifluormethoxy-4,6-difluor-benzyl-2,2-dimethyl-3-(2,2-dichlorvinyl)-cyclopropancarboxylat,
3-Trifluormethoxy-4,6-difluor-benzyl-2,2-dimethyl-3-(2-chlor-3,3,3-trifluor-1-propenyl)-cyclopropancarboxylat,
3-Trifluormethoxy-4,6-difluor-benzyl-2,2-dimethyl-3-(2-chlor-3,3,4,4,4-pentafluor-1-butenyl)-cyclopropancarboxylat,
3-Trifluormethoxy-2,4,6-trifluor-benzyl-2,2-dimethyl-3-(2,2-dichlorvinyl)-cyclopropancarboxylat,
3-Trifluormethoxy-2,4,6-trifluor-benzyl-2,2-dimethyl-3-(2-chlor-3,3,3-trifluor-1-propenyl)-cyclopropancarboxylat,
3-Trifluormethoxy-2,4,6-trifluor-benzyl-2,2-dimethyl-3-(2-chlor-3,3,4,4,4-pentafluor-1-butenyl)-cyclopropancarboxylat,
3-Trifluormethoxy-2,4,6-trifluor-5-chlor-benzyl-2,2-dimethyl-3-(2,2-dichlorvinyl)-cyclopropancarboxylat,
3-Trifluormethoxy-2,4,6-trifluor-5-chlor-benzyl-2,2-dimethyl-3-(2-chlor-3,3,3-trifluor-1-propenyl)-cyclopropancarboxylat,
3-Trifluormethoxy-2,4,6-trifluor-5-chlor-benzyl-2,2-dimethyl-3-(2-chlor-3,3,4,4,4-pentafluor-1-butenyl)-cyclopropancarboxylat,
3-Trifluormethoxy-4-fluor-5-chlor-benzyl-2,2-dimethyl-3-(2,2-dichlorvinyl)-cyclopropan-carboxylat,
3-Trifluormethoxy-4-fluor-5-chlor-benzyl-2,2-dimethyl-3-(2-chlor-3,3,3-trifluor-1-propenyl)-cyclopropancarboxylat,
3-Trifluormethoxy-4-fluor-5-chlor-benzyl-2,2-dimethyl-3-(2-chlor-3,3,4,4,4-pentafluor-1-butenyl)-cyclopropancarboxylat,
3-Trifluormethoxy-5-chlor-6-fluor-benzyl-2,2-dimethyl-3-(2,2-dichlorvinyl)-cyclopropancarboxylat,
3-Trifluormethoxy-5-chlor-6-fluor-benzyl-2,2-dimethyl-3-(2-chlor-3,3,3-trifluor-1-propenyl)-cyclopropancarboxylat,
3-Trifluormethoxy-5-chlor-6-fluor-benzyl-2,2-dimethyl-3-(2-chlor-3,3,4,4,4-pentafluor-1-butenyl)-cyclopropancarboxylat,

Die Herstellung der erfindungsgemäßen Benzylester der Formel (I) kann durch folgendes Reaktions-

schema wiedergegeben werden:

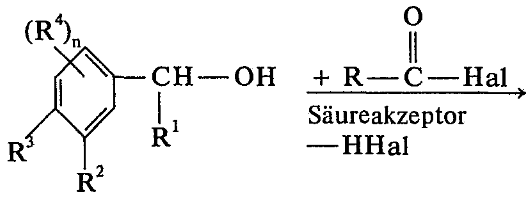


(I)

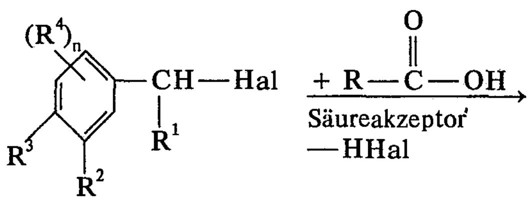


(I)

Verwendet man beispielsweise gemäß Verfahren 2 (oben) 2,2-Dimethyl-3-(2,2-dichlorvinyl)-cyclopropancarbonsäurechlorid und 3-Trifluormethoxy-6-fluor-benzylalkohol als Ausgangsmaterialien, so kann der Reaktionsablauf durch das folgende Formelschema wiedergegeben werden:

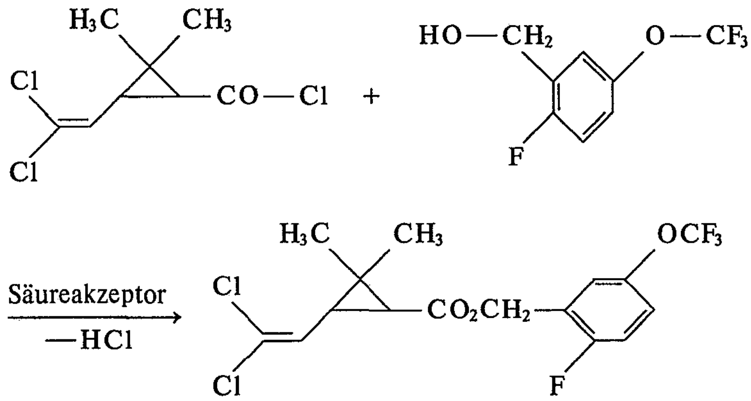


Verwendet man ein reaktionsfähiges Derivat des Alkohols der allgemeinen Formel (III), so kommen insbesondere die Benzylhalogenide der Formel (V) in Betracht.

Verwendet man beispielsweise 2,2-Dimethyl-3-(2-chlor-3,3,4,4,4-pentafluor-1-butenyl)-cyclopropancarbonsäure und 3-Trifluormethoxy-4-fluorbenzylbromid als Ausgangsmaterialien, so kann der Reaktionsablauf durch das folgende Formelschema wiedergegeben werden:

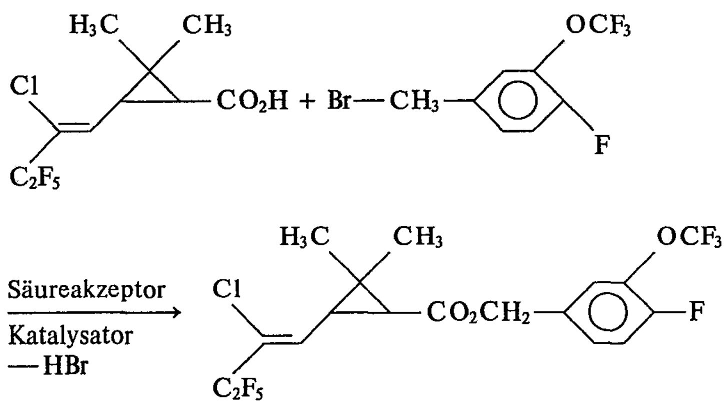


Die als Ausgangsprodukte zu verwendenden Carbonsäuren bzw. reaktionsfähigen Derivate derselben der Formel (II) sind bekannt und nach in der Literatur beschriebenen Verfahren herstellbar (vgl. z. B. DE-OS 2 326 077, 2 800 922, 2 802 962).

Bevorzugt sind Verbindungen der Formel (II) in welcher R die weiter oben angegebene bevorzugte oder besonders bevorzugte Bedeutung hat.

Die allgemeine Formel (II) schließt dabei die verschiedenen Stereoisomeren, die optischen Isomeren und Mischungen dieser Komponenten ein.

Als Beispiele für die als Ausgangsprodukte zu verwendenden Verbindungen der Formel (II) seien im einzelnen genannt:

2,2-Dimethyl-3-(2,2-dichlorvinyl)-cyclopropancarbonsäure,
2,2-Dimethyl-3-(2-chlor-3,3,3-trifluor-1-propenyl)-cyclopropancarbonsäure,
2,2-Dimethyl-3-(2-chlor-3,3,4,4,4-pentafluor-1-butenyl)-cyclopropancarbonsäure,
2,2-Dimethyl-3-(2,2-difluorvinyl)-cyclopropancarbonsäure,
2,2-Dimethyl-3-(2,2-dibromvinyl)-cyclopropancarbonsäure,
2,2-Dimethyl-3-(2-methyl-1-propenyl)-cyclopropancarbonsäure,
2,2-Dimethyl-3-(2-trifluormethyl-3,3,3-trifluor-1-propenyl)-cyclopropancarbonsäure.

Die ebenfalls als Ausgangsstoffe zu verwendenden Alkohole der Formel (III) sind neu.
Die neuen Alkohole können nach den unter 4. (oben) angegebenen Verfahren hergestellt werden (Einzelheiten weiter unten).
Bevorzugt werden Alkohole der Formel (III) verwendet, in welcher $R^1$, $R^2$, $R^3$, $R^4$ und n die weiter oben angegebene bevorzugte oder besonders bevorzugte Bedeutung haben.
Als Beispiele für die als Ausgangsprodukte zu verwendenden Alkohole der Formel (III) seien im einzelnen genannt:

Difluor-3,4-dioxymethylen-6-fluor-benzylalkohol,
3-Trifluormethoxy-2-fluor-benzylalkohol,
3-Trifluormethoxy-4-fluor-benzylalkohol,
3-Trifluor-5-fluor-benzylalkohol,
3-Trifluormethoxy-6-fluor-benzylalkohol,
3-Trifluormethoxy-4-fluor-5-chlor-benzylalkohol,
3-Trifluormethoxy-2,4,6-trifluor-benzylalkohol,
3-Trifluormethoxy-2,4,6-trifluor-5-chlor-benzylalkohol,
3-Trifluormethoxy-5-chlor-6-fluor-benzylalkohol,
3-Trifluormethoxy-4,6-difluor-benzylalkohol.

Zur Herstellung der erfindungsgemäßen Verbindungen der Formel (I) gemäß 1. (oben) aus Alkoholen (oder einem reaktionsfähigen Derivat derselben) der Formel (III) und Carbonsäuren (bzw. reaktionsfähige Derivate derselben) der Formel (II) können als Säureakzeptoren alle üblichen Säurebindemittel Verwendung finden.
Besonders bewährt haben sich bei Einsatz der Säurehalogenide als reaktionsfähige Derivate Alkalicarbonate und -alkoholate, wie Natrium- und Kaliumcarbonat, Natrium- und Kaliummethylat bzw. -ethylat, ferner aliphatische, aromatische oder heterocyclische Amine, beispielsweise Triethylamin, Trimethylamin, Dimethylamin, Dimethylbenzylamin und Pyridin.
Die Reaktionstemperaturen können innerhalb eines größeren Bereiches variiert werden. Im allgemeinen arbeitet man zwischen 0 und 100°C, vorzugsweise bei 15 bis 40°C.
Die Reaktion läßt man im allgemeinen bei Normaldruck ablaufen. Das Verfahren zur Herstellung der erfindungsgemäßen Verbindungen wird bevorzugt unter Mitverwendung geeigneter Lösungs- und Verdünnungsmittel durchgeführt. Als solche kommen praktisch alle inerten organischen Solventien infrage. Hierzu gehören insbesondere aliphatische und aromatische, gegebenenfalls chlorierte Kohlenwasserstoffe, wie Benzol, Toluol, Xylol, Benzin, Methylenchlorid, Chloroform, Tetrachlorkohlenstoff, z. B. Diethyl- und Dibutylether, Dioxan, ferner Ketone, beispielsweise Aceton, Methylethyl-, Methylisopropyl- und Methylisobutylketon, außerdem Nitrile, wie Aceto- und Propionitril.
Zur Durchführung des Verfahrens setzt man die Ausgangskomponenten vorzugsweise in äquimolaren Verhältnissen ein. Ein Überschuß der einen oder anderen Komponente bringt keine wesentlichen Vorteile. Die Reaktionskomponenten werden im allgemeinen in einem der angegebenen Lösungsmittel zusammen gegeben und meist bei erhöhter Temperatur zur Vervollständigung der Reaktion eine oder mehrere Stunden gerührt. Anschließend gießt man das Reaktionsgemisch in Wasser, trennt die organische Phase ab und wäscht diese mit Wasser nach. Nach dem Trocknen wird das Lösungsmittel im Vakuum abdestilliert.
Die neuen Verbindungen fallen in Form von Ölen an, die sich zum Teil nicht unzersetzt destillieren lassen, jedoch durch sogenanntes »Andestillieren« d. h. durch längeres Erhitzen unter vermindertem Druck auf mäßig erhöhte Temperaturen von den letzten flüchtigen Anteilen befreit und auf diese Weise gereinigt werden. Zu ihrer Charakterisierung dient der Brechungsindex.
Verwendet man als reaktionsfähiges Derivat der Alkohole die Benzylhalogenide der Formel (V) (oben), können als Säureakzeptoren ebenfalls alle üblichen Säurebindemittel Verwendung finden. Besonders bewährt haben sich die Alkalihydroxide, wie Natrium- oder Kaliumhydroxid, oder aber Amine, wie Triethylamin.
Die Reaktionstemperatur liegt zwischen 50 und 150°C, vorzugsweise zwischen 80 und 130°C.
Als Lösungsmittel seien Aceton, Acetonitril, Benzol, Toluol, Xylol, Chlorbenzol und Dichlorbenzol genannt.

Die Reaktion wird vorzugsweise in Gegenwart von Phasentransferkatalystoren, wie beispielsweise quartären Ammoniumsalzen, durchgeführt. Es seien beispielsweise Tetrabutylammoniumchlorid oder -bromid, Benzyltriethylammoniumchlorid oder Methyltrioctylammoniumchlorid genannt.

Zur Durchführung des Verfahrens setzt man die Ausgangskomponenten vorzugsweise in äquimolaren Verhältnissen ein. ein Überschuß der Säure und des Säureakzeptors kann verwendet werden, um eine vollständige Umsetzung des Benzylhalogenids zu erreichen. Die überschüssige Säure kann aus der Wasserphase wiedergewonnen werden. Die Reaktion ist meist nach 1—5 Stunden beendet. Nach Abkühlen des Reaktionsgemisches wird mit Wasser versetzt, die organische Phase abgetrennt und neutral gewaschen. Anschließend wird das Lösungsmittel im Vakuum abdestilliert und die Verbindungen der Formel (I) wie oben beschrieben gereinigt.

Wie bereits erwähnt sind die Alkohle der Formel (III) neu. Sie lassen sich nach den unter 4. (oben) angegebenen Verfahren herstellen.

Bei Variante 4a wird für den Fall, daß in der gewünschten Verbindung der Formel (III) $R^1$ für Wasserstoff steht der entsprechende Aldehyd mit Wasserstoff reduziert. Dies läßt sich durch folgendes Formelschema darstellen:

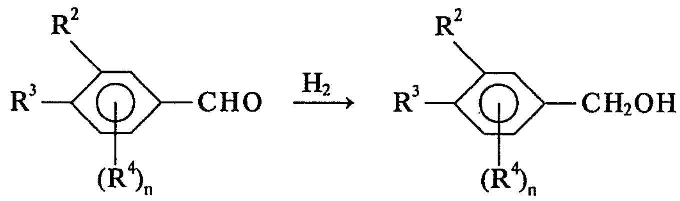


Als Reduktionsmittel kommen Wasserstoff in Anwesenheit von Katalysatoren oder komplexe Metallhydride wie beispielsweise Natriumborhydrid oder Lithiumaluminiumhydrid infrage. Die Durchführung der Reaktion erfolgt analog zu bekannten Verfahren (vgl.: Organikum, VEB Deutscher Verlag der Wissenschaften, Berlin 1965, 5. Auflage, Seite 417; J. Am. Chem. Soc. 71, 122 (1949); 75, 199 (1953) und 76, 6116 (1954)).

Für den Fall, daß in der gewünschten Verbindung der Formel (III) $R^1$ für CN steht wird der entsprechende Aldehyd mit HCN umgesetzt. Dies läßt sich durch folgendes Formelschema darstellen:

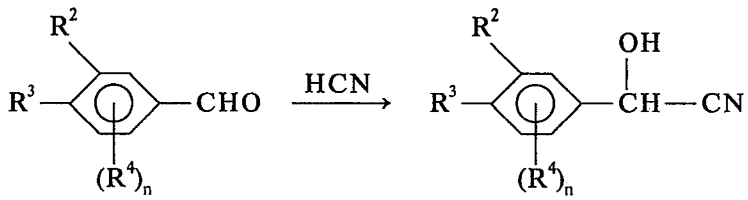


Die Durchführung der Reaktion erfolgt analog den bekannten Verfahren zur Herstellung von Cyanhydrinen (vgl. Organic Synthesis; Coll. Vol. I, 336; Houben-Weyl, Band VIII, Seite 274 f.).

Bei Verfahren 4a werden bevorzugt Aldehyde der Formel (IV) eingesetzt, in welcher $R^2$—$R^4$ und n die weiter oben angegebene bevorzugte oder besonders bevorzugte Bedeutung besitzen.

Folgende Aldehyde der Formel (IV) seien im einzelnen genannt:

3-Trifluormethoxy-4-fluor-benzaldehyd
3-Trifluormethoxy-2-fluor-benzaldehyd

Bei Variante 4b werden die Benzylhalogenide der Formel (V) verseift. Diese Reaktion läßt sich durch folgendes Formelschema darstellen:

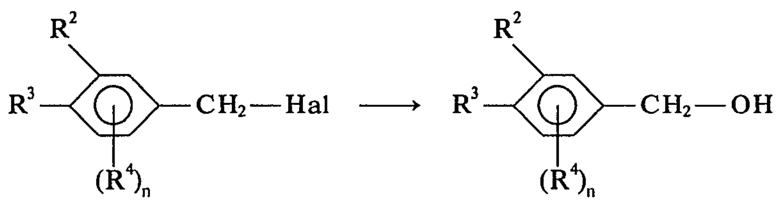

Die Verseifung erfolgt dabei in an sich bekannter Weise mit wäßrigen Basen, wie beispielsweise NaOH, KOH oder Alkalicarbonate, wie $Na_2CO_3$ oder $K_2CO_3$.

Folgende Benzylhalogenide der Formel (V) seien im einzelnen genannt:

3-Trifluormethoxy-4-fluorbenzylchlorid und -bromid

3-Trifluormethoxy-2-fluorbenzylchlorid und -bromid
3-Trifluormethoxy-6-fluorbenzylchlorid und -bromid
Difluor-3,4-dioxymethylen-6-fluor-benzylchlorid und -bromid
3-Trifluormethoxy-5-chlor-6-fluorbenzylchlorid und -bromid

Bei Verfahren 4c werden die Säurehalogenide der Formel (VI) mit einem Alkalicarbonat, wie Natrium- oder Kaliumboranat reduziert.

Diese Reaktion erfolgt in an sich bekannter Weise und läßt sich durch folgendes Formelschema darstellen:

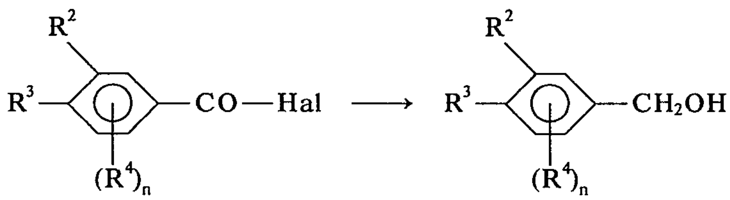

Folgende Benzylhalogenide der Formel (VI) seien im einzelnen genannt:

3-Trifluormethoxy-4-fluorbenzoylfluorid
3-Trifluormethoxy-4-fluor-5-chlor-benzoylfluorid
3-Trifluormethoxy-2-fluor-benzoylfluorid
3-Trifluormethoxy-2,4,6-trifluor-benzoylchlorid
3-Trifluormethoxy-2,4,6-trifluor-5-chlor-benzoylfluorid
3-Trifluormethoxy-4,6-difluor-benzoylfluorid

Die neuen Aldehyde der Formel (IV) werden nach Verfahren 6a (oben) dadurch erhalten, daß man Benzylhalogenide der Formel (V) mit Urotropin umsetzt und anschließend hydrolysiert.

Diese Reaktion erfolgt in an sich bekannter Weise (»Sommelet-Reaktion«, Organic Syntheses, Coll. Vol. II, S. 336, III, S. 811, IV, 690, 918, 932). Sie läßt sich durch folgendes Formelschema darstellen:

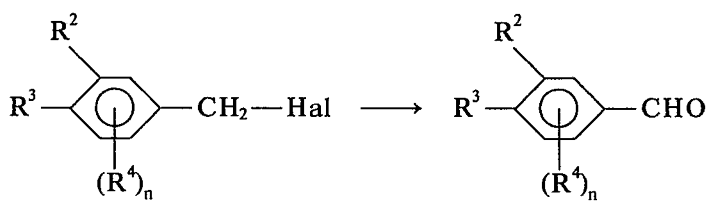

Nach Verfahren 6b setzt man die Benzylchloride oder -bromide der Formel (VI) mit Trialkylphosphit, wie beispielsweise Trimethyl- oder Triethylphosphit in an sich bekannter Weise um (Houben—Weyl), Band 12, 1, Seite 453) und reduziert anschließend in an sich bekannter Weise mit Alkaliborhydriden, wie beispielsweise Natriumborhydrid (Chemische Berichte 103, S. 2984, 1970).

Die neuen Benzylhalogenide der Formel (V) erhält man gemäß Verfahren 8. (oben), indem man nach im Prinzip bekannten Methoden Verbindungen der Formel (VII) radikalisch halogeniert, insbesondere bromiert oderr chloriert.

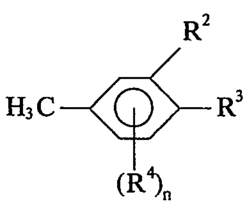

(VII)

Als Halogenierungsmittel kommen beispielsweise Chlor oder N-Chlor- bzw. N-Brom-succinimid infrage.

Die Chlorierung oder Bromierung der vorgenannten Verbindungen zu den entsprechenden Benzylhalogeniden oder -bromiden erfolgt in an sich bekannter Weise unter radikalischen Bedingungen mit Chlor, N-Chlorsuccinimid oder N-Brom-succinimid in Lösungsmitteln wie beispielsweise Methylenchlorid, Chloroform, Tetrachlorkohlenstoff, Chlorbenzol, o-Difluorbenzol, vorzugsweise bei erhöhter Temperatur.

Bevorzugte Ausgangsverbindungen der Formel (VII) sind solche in welcher $R^2$—$R^4$ und n für die oben angegebenen bevorzugten oder besonders bevorzugten Definitionen steht. Als einzelne Verbindungen seien genannt:

3-Trifluormethoxy-2-fluor-toluol
3-Trifluormethoxy-4-fluor-toluol
3-Trifluormethoxy-6-fluor-toluol
Difluor-3,4-methylendioxy-6-fluor-toluol
3-Trifluormethoxy-5-chlor-6-fluor-toluol.

Die neuen Benzoylhalogenide der Formel (VI) erhält man gemäß Verfahren 9 (oben), indem man nach im Prinzip bekannten Methoden Verbindungen der Formel (VIII) mit einem Alkalifluorid, vorzugsweise Kaliumfluorid, in einem geeigneten hochsiedenden Lösungsmittel, beispielsweise Sulfolan, umsetzt.
Verwendet man bei Verfahren 9 beispielsweise 3-Trifluormethoxy-4-chlor-benzoylchlorid als Ausgangsstoff, so kann der Reaktionsablauf durch folgendes Formelschema wiedergegeben werden.

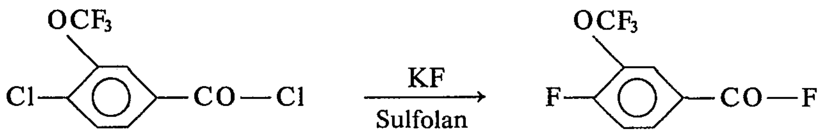


Da für Verfahren 6b die Säurefluoride nicht eingesetzt werden können, muß man entweder zur Säure verseifen und in das Säurechlorid oder -bromid überführen oder aber direkt, beispielsweise mit Bortrichlorid, das Säurechlorid herstellen.
Die Ausgangsstoffe der Formel (VIII) sind bekannt oder können nach bekannten Verfahren hergestellt werden (DE-OS 2 819 788).
Als einzelne Verbindungen seien genannt:

3-Trifluormethoxy-4-chlor-benzoylfluorid und -chlorid
3-Trifluormethoxy-4,5-dichlorbenzoylfluorid und -chlorid
3-Trifluormethoxy-4,6-dichlorbenzoylfluorid und -chlorid
3-Trifluormethoxy-2,4,6-trichlorbenzoylfluorid und -chlorid
3-Trifluormethoxy-2,4,5,6-tetrachlor-benzoylfluorid und -chlorid
Difluor-3,4-dioxymethylen-6-chlor-benzoylfluorid und -chlorid.

Die Toluole der Formel (VII) erhält man gemäß Verfahren 12a (oben), indem man nach im Prinzip bekannten Methoden Verbindungen der Formel (IX) mit Nitrosyltetrafluoroborat umsetzt (»Schiemann-Reaktion«, Organic Syntheses Coll. Vol. II, 188, 295, 299) und dadurch die Aminogruppe durch ein Fluoratom ersetzt.
Verwendet man bei Verfahren 12a beispielsweise 3-Trifluormethoxy-5-amino-toluol, so kann der Reaktionsverlauf durch folgendes Formelschema wiedergegeben werden:

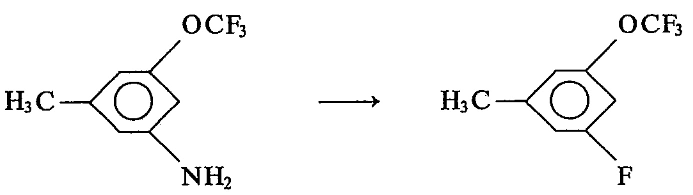

Folgende Aniline der Formel (IX) seien im einzelnen genannt:

3-Trifluormethoxy-5-methyl-anilin
4-Trifluormethoxy-2-methyl-anilin
4-Trifluormethoxy-2-methyl-5-fluor-anilin.

Toluole der Formel (VII) erhält man außerdem gemäß Verfahren 12b (oben), indem man nach einer im Prinzip bekannten Methode (US-Patent 4 157 344) die Phenole der Formel (X) mit Fluorwasserstoff in Gegenwart von Tetrachlorkohlenstoff umsetzt.
Die Phenole der Formel (X) sind bekannt und können nach bekannten Methoden erhalten werden (J. Am. Chem. Soc. 81, S. 94, 1959; Houben—Weyl, Band 6lc, Seite 166 und dort angegebene Literatur).
Folgende Phenole der Formel (X) seien im einzelnen genannt:

3-Methyl-6-fluor-phenol
3-Methyl-4-fluor-phenol
3-Methyl-5-fluor-phenol.

Die Aniline der Formel (IX) (vgl. 13, oben) erhält man nach Verfahren 14a, oben), indem man nach im

Prinzip bekannter Weise (vgl. US-Patent 4 157 344) Hydroxyaniline der Formel (XI) mit Fluorwasserstoff in Gegenwart von Tetrachlorkohlenstoff umsetzt.

Die Hydroxyaniline der Formel (XI) sind literaturbekannt.

Folgende Hydroxyaniline seien im einzelnen genannt:

2-Methyl-4-hydroxy-anilin
5-Methyl-3-hydroxy-anilin

Für den Fall, daß $R^2$ und $R^3$ gemeinsam fluorsubstituiertes Dioxymethylen bedeuten, erhält man das Anilin der Formel (IX) gemäß Verfahren 14b (oben), indem man in an sich bekannter Weise die Verbindung (XII) nitriert und reduziert:

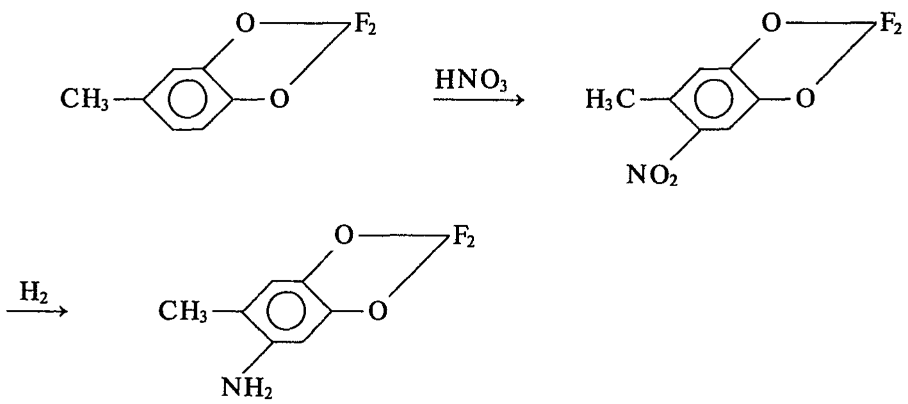


Die Wirkstoffe eignen sich bei guter Pflanzenverträglichkeit und günstiger Warmblütertoxizität zur Bekämpfung von tierischen Schädlingen, insbesondere Insekten und Spinnentieren, die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:

Aus der Ordnung der Isopoda z. B. Oniscus asellus, Armadillidium vulgare, Porcellio scaber.
Aus der Ordnung der Diplopoda z. B. Blaniulus guttulatus.
Aus der Ordnung der Chilopoda z. B. Geophilus carpophagus, Scutigera spec.
Aus der Ordnung der Symphyla z. B. Scutigerella immaculata.
Aus der Ordnung der Thysanura z. B. Lepisma saccharina.
Aus der Ordnung der Collembola z. B. Onychiurus armatus.
Aus der Ordnung der Orthoptera z. B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.
Aus der Ordnung der Dermaptera z. B. Forficula auricularia.
Aus der Ordnung der Isoptera z. B. Reticulitermes spp.
Aus der Ordnung der Anoplura z. B. Phylloxera vastatrix, Pemphigus spp., Pediculus humanus corporis, Haematopinus spp., Linognathus spp.
Aus der Ordnung der Mallophaga z. B. Trichodectes spp., Damalinea spp.
Aus der Ordnung der Thysanoptera z. B. Hercinothrips femoralis, Thrips tabaci.
Aus der Ordnung der Heteroptera z. B. Eurygaster spp., Dysdercus intermedius, Piesma quadrata, Cimex lectularius, Rhodnius prolixus, Triatoma spp.
Aus der Ordnung der Homoptera z. B. Aleurodes brassicae, Bemisia tabaci, Trialeurodes vaporariorum, Aphis gossypii, Brevicoryne brassicae, Cryptomyzus ribis, Doralis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp.
Aus der Ordnung der Ledidoptera z. B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella, Agrotis spp., Euxoa spp., Feltia spp. Earias insulana, Heliothi spp., Laphygma exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Cacoecia podana, Capua reticulana, Choristoneura fumiferana, Clysia ambiguella, Homona magnanima, Tortrix viridana.
Aus der Ordnung der Coleoptera z. B. Anobium punctatum, Rhizopertha dominica, Bruchidius

obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermetes spp., Trogoderma spp., Anthrenus spp., Attagenus spp., Lyctus spp., Meligethes aeneus, Ptinus spp., Niptus hololeucus Gibbium psylloides, Tribolium spp., Tenebrio molitor, Agriotes spp., Conoderus spp., Melolontha melolontha, Amphimallon solstitialis, Costelytra zealandica.
Aus der Ordnung der Hymenoptera z. B. Diprion spp., Hoplocampa spp., Lasius spp., Monomorium pharaonis, Vespa spp.
Aus der Ordnung der Diptera z. B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.
Aus der Ordnung der Siphonaptera z. B. Xenopsylla cheopis, Ceratophyllus spp.
Aus der Ordnung der Arachnida z. B. Scorpio maurus, Latrodectus mactans.
Aus der Ordnung der Acarina z. B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp.

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen, sowie ULV-Kalt- und Warmnebel-Formulierungen.

Diese Formulierungen werden in bekannter Weise hergestellt, z. B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z. B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chloräthylene oder Methylenchlorid, aliphatische Kohlenwasserstoffe, wie Cyclohexan oder Paraffine, z. B. Erdölfraktionen, Alkohole, wie Butanol oder Glycol sowie deren Äther und Ester, Ketone, wie Aceton, Methyläthylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z. B. Aerosol-Treibgas, wie Halogenkohlenwasserstoffe sowie Butan, Propan, Stickstoff und Kohlendioxid; als feste Trägerstoffe kommen in Frage: z. B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z. B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z. B. nichtionogene und anionische Emulgatoren, wie Polyoxyäthylen-Fettsäure-Ester, Polyoxyäthylen-Fettalkohol-Äther, z. B. Alkylarylpolyglykol-äther, Alkylsulfonate, Alkylsulfate, Arylsulfonate sowie Eiweißhydrolysate; als Dispergiermittel kommen in Frage: z. B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z. B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90%.

Die erfindungsgemäßen Wirkstoffe können in ihren handelsüblichen Formulierungen sowie in den aus diesen Formulierungen bereiteten Anwendungsformen in Mischung mit anderen Wirkstoffen, wie Insektiziden, Lockstoffen, Sterilantien, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe.

Die erfindungsgemäßen Wirkstoffe können ferner in ihren handelsüblichen Formulierungen sowie in den aus diesen Formulierungen bereiteten Anwendungsformen in Mischung mit Synergisten vorliegen.

Synergisten sind Verbindungen, durch die die Wirkung der Wirkstoffe gesteigert wird, ohne daß der zugesetzte Synergist selbst aktiv wirksam sein muß.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 100 Gew.-% Wirkstoff, vorzugsweise zwischen 0,0001 und 1 Gew.-% liegen.

Die Anwendung geschieht in einer den Anwendungsformen angepaßten üblichen Weise.

Bei der Anwendung gegen Hygiene- und Vorratsschädlinge zeichnen sich die Wirkstoffe durch eine hervorragende Residualwirkung auf Holz und Ton sowie durch eine gute Alkalistabilität auf gekälkten Unterlagen aus.

Die erfindungsgemäßen Wirkstoffe eignen sich auch zur Bekämpfung von Ekto- und Endoparasiten auf dem veterinärmedizinischen Gebiet.

Die Anwendung der erfindungsgemäßen Wirkstoffe geschieht im Veterinärsektor in bekannter Weise, wie durch orale Anwendung in Form von beispielsweise Tabletten, Kapseln, Tränken, Granulaten, durch dermale Anwendung in Form beispielsweise des Tauchens (Dippen), Sprühens (Sprayen), Aufgießen (pour-on and spot-on) und des Einpuderns sowie durch parenterale Anwendung in Form beispielsweise der Injektion.

## Herstellungsbeispiele

### Beispiel 1

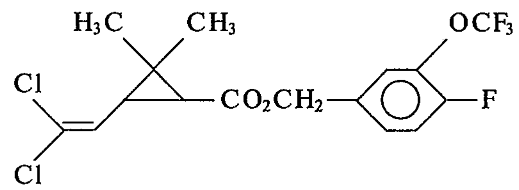

16 g (0,07 Mol) 2,2-Dimethyl-3-(2,2-dichlorvinyl)-cyclopropancarbonsäurechlorid, (±)-cis/trans, werden in 100 ml Toluol gelöst und 15 g 3-Trifluormethoxy-4-fluor-benzylalkohol zugetropft. Anschließend werden bei 20–25°C 5,5 g Pyridin in 50 ml Toluol gelöst zugetropft. Man rührt 3 Stunden bei Raumtemperatur nach, gießt das Reaktionsgemisch in 150 ml Wasser, trennt die Toluolphase ab und wäscht sie mit 100 ml Wasser, trocknet mit Natriumsulfat und destilliert das Toluol im Vakuum ab. Letzte Lösungsmittelreste werden durch Andestillieren bei einer Badtemperatur 60°C/0,2 bis 1,0 Torr entfernt. Man erhält 22,8 g 3-Trifluormethoxy-4-fluor-benzyl-2,2-dimethyl-3-(2,2-dichlorvinyl)-cyclopropancarboxylat als viskoses Öl vom Brechungsindex $n_D^{20} = 1{,}503$.

### Beispiel 2

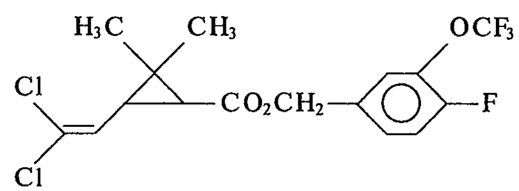

Es wird analog Beispiel 1 gearbeitet, jedoch wird als Säurekomponente (+)-trans-2,2-Dimethyl-3-(2,2-dichlorvinyl)-cyclopropancarbonsäure verwendet. ($[\alpha]_{20}^{D}$: 4,0° ($CHCl_3$) Mittelwert).

### Beispiel 3

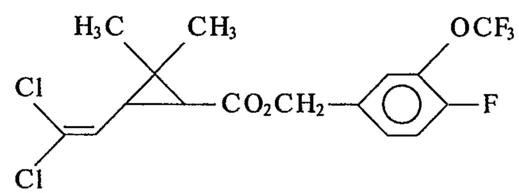

Es wird analog Beispiel 1 gearbeitet, jedoch wird als Säurekomponente (+)-cis-2,2-Dimethyl-3-(2,2-

dichlorvinyl)-cyclopropancarbonsäure verwendet. $[\alpha]_{20}^{D} = 12,3°$ ($CHCl_3$; Mittelwert).
Analog Beispiel 1 wurden ferner erhalten:

| Beispiel | Formel | Brechungs-index ($n_D^{20}$) |
|---|---|---|
| 4 | 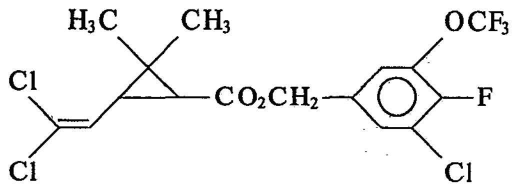 | 1,499 |
| 5 | 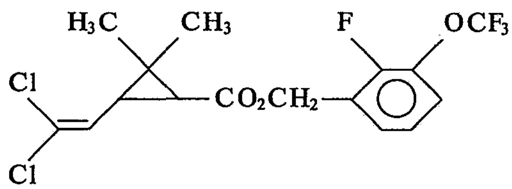 | 1,400 |
| 6 | 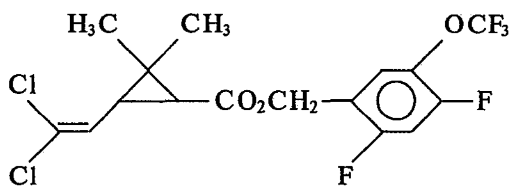 | 1,487 |
| 7 | 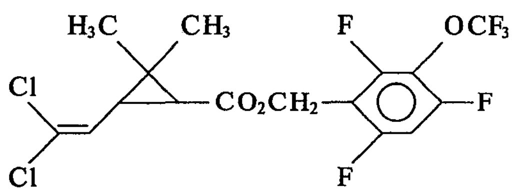 | 1,491 |
| 8 | 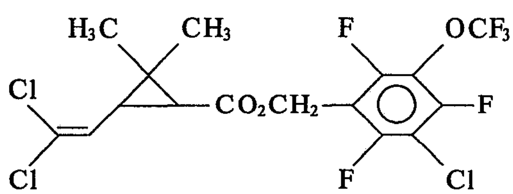 | 1,502 |
| 9 | 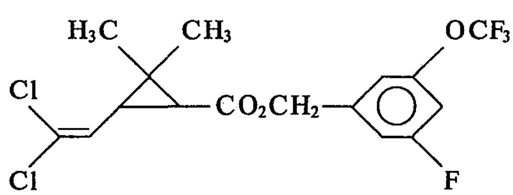 | 1,477 |

Beispiel 10

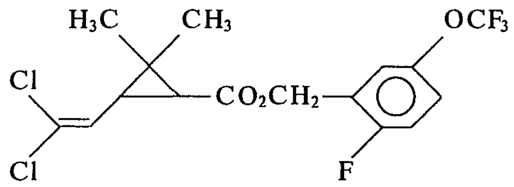

Man mischt 10 g 2,2-Dimethyl-3-(2,2-dichlorvinyl)-cyclopropancarbonsäure ((±)-trans), 10,9 g 3-Trifluormethoxy-6-fluor-benzylbromid, 100 ml Toluol, 3,1 g pulverisierte KOH (technisch 87%ig) und 1 g Tetrabutylammoniumbromid und erhitzt 4 Stunden zum Sieden. Nach dem Abkühlen gießt man das Reaktionsgemisch in 150 ml Wasser, trennt die Toluolphasse ab, wäscht sie mit Bicarbonatlösung und anschließend zweimal mit Wasser. Nach Trocknen mit Natriumsulfat destilliert man das Toluol im Vakuum ab. Letzte Lösungsmittelreste werden durch Andestillieren bei einer Badtemperatur von 60—100°C/0,2 bis 1,0 Torr entfernt. Man erhält 16,3 g 3-Trifluormethoxy-6-fluor-benzyl-trans-2,2-dimethyl-3-(2,2-dichlorvinyl)-cyclopropancarboxylat als viskoses Öl mit dem Brechungsindex $n_D^{20}$ = 1,483.

Beispiel 11

Analog Beispiel 10 wird der entsprechende Ester aus 3-Trifluormethoxy-6-fluor-benzylbromid und der (+)-trans- sowie der (—)-trans-2,2-Dimethyl-3-(2,2-dichlorvinyl)-cyclopropancarbonsäure erhalten.

Der Ester mit der (—)-trans-Säure ist bedeutend schwächer wirksam.

Beispiel 12

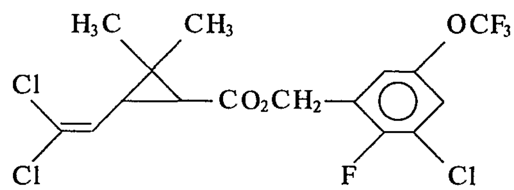

Aus 3-Trifluormethoxy-5-chlor-6-fluor-benzylbromid und (±)-cis/trans-2,2-Dimethyl-3-(2,2-dichlorvinyl)-cyclopropancarbonsäure erhält man analog Beispiel 10 3-Trifluormethoxy-5-chlor-6-fluor-benzyl-2,2-dimethyl-3-(2,2-dichlorvinyl)-cyclopropancarboxylat als viskoses Öl mit dem Brechungsindex $n_D^{20}$ = 1,498.

Beispiel 13

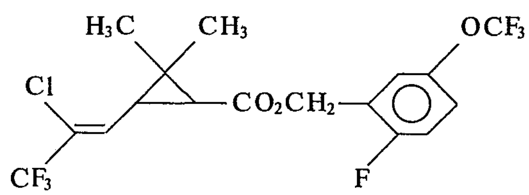

Analog Beispiel 10 werden aus 9,5 g 2,2-Dimethyl-3-(2-chlor-3,3,3-trifluor-1-propenyl)-cyclopropancarbonsäure, 9,6 g 3-Trifluormethoxy-6-fluor-benzylbromid, 2,5 g KOH (pulverisiert, 87%ig) und 1 g Tetrabutylammoniumbromid 13 g 3-Trifluormethoxy-6-fluor-benzyl-2,2-dimethyl-3-(2,2-dichlorvinyl)-cyclopropancarboxylat erhalten. Brechungsindex $n_D^{20}$ = 1,452.

Analog wurden erhalten:

| Beispiel | Formel | Brechungs-index ($n_D^{20}$) |
| --- | --- | --- |
| 14 | 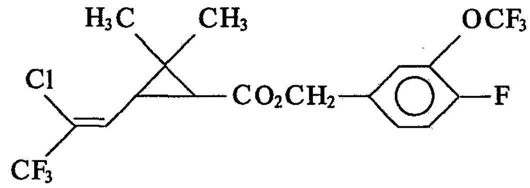 | 1,455 |
| 15 | 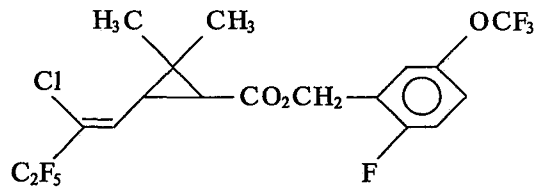 | 1,441 |
| 16 | 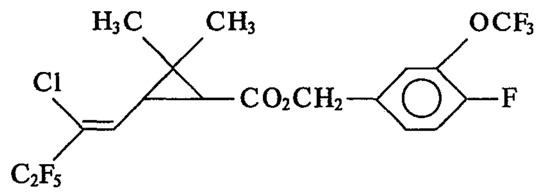 | 1,446 |
| 17 | 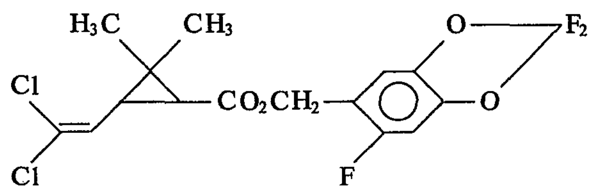 | 1,496 |
| 18 | 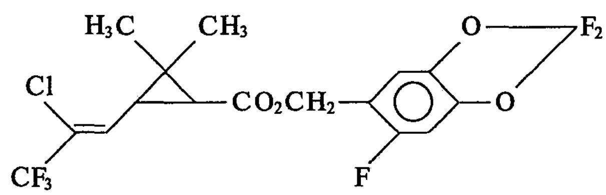 | 1,464 |

## Beispiel 19

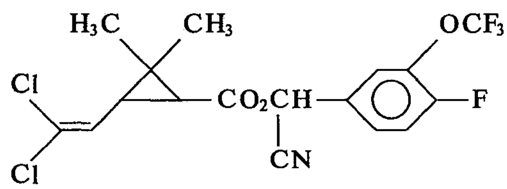

Analog Beispiel 1 erhält man aus 2,2-Dimethyl-3-(2,2-dichlorvinyl)-cyclopropancarbonsäurechlorid und 3-Trifluormethoxy-4-fluor-$\alpha$-cyano-benzylalkohol 3-Trifluormethoxy-4-fluor-$\alpha$-cyano-benzyl-2,2-dimethyl-3-(2,2-dichlorvinyl)-cyclopropancarboxylat als viskoses Öl mit dem Brechungsindex $n_D^{20} = 1,491$.

Analog wurden erhalten:

| Beispiel | Formel | Brechungsindex $n_D^{20}$ bzw. Drehwinkel $[\alpha]_{20}^{D}$ |
|---|---|---|
| 20 | 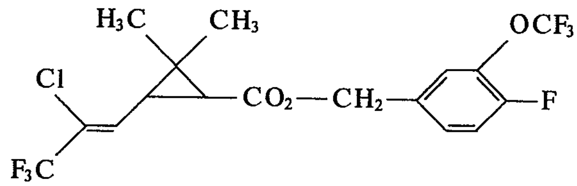 Säure: (+)-trans | 1,454<br>−11,5° ($CHCl_3$) |
| 21 | 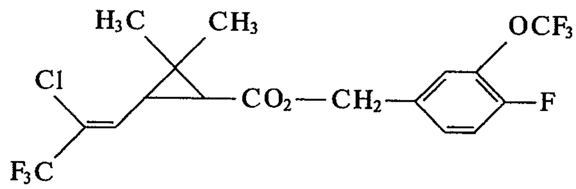 Säure: (−)-trans | 1,454<br>−18,4° ($CHCl_3$) |
| 22 | 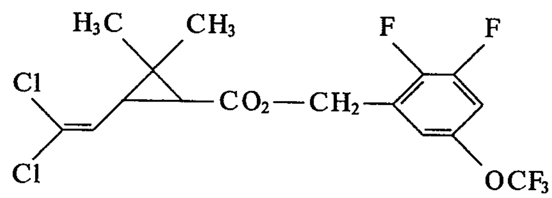 (±) cis/trans | 1,482 |
| 23 | 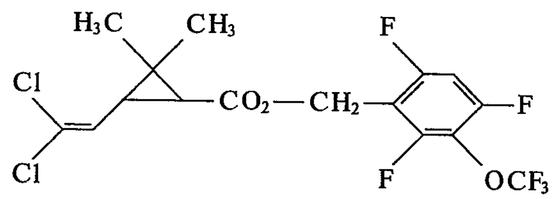 Säure: (+)-trans | 1,486<br>−4,6° ($CHCl_3$) |
| 24 | 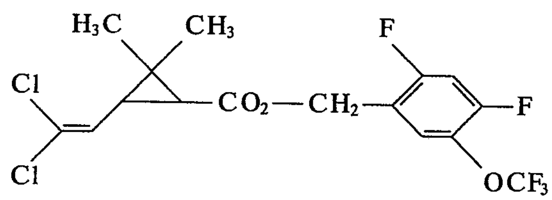 Säure: (+)-trans | 1,483<br>−3,9° ($CHCl_3$) |

Fortsetzung

| Beispiel | Formel | Brechungsindex $n_D^{20}$ bzw. Drehwinkel $[\alpha]_{20}^D$ |
|---|---|---|
| 25 | 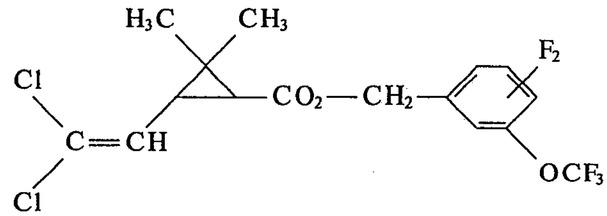 (25) (+)-trans | |

## Herstellung der Ausgangsstoffe

### A. Herstellung der Benzylalkohole (III)

#### 3-Trifluormethoxy-4,6-difluor-benzylalkohol

3 g Natriumborhydrid werden in 70 ml trockenem Dioxan bei 20°C unter Rühren vorgelegt und 10 g 3-Trifluormethoxy-4,6-difluor-benzoylfluorid in 20 ml Dioxan zugetropft. Man rührt zuerst 2 Stunden bei 20°C und anschließend 1 Stunde bei Rückfluß. Nach dem Abkühlen auf 20°C werden 20 ml Wasser zugetropft und dann durch 20%ige Schwefelsäure sauer gestellt. Die Reaktionsmischung wird mit Methylenchlorid extrahiert und nach Abdampfen des Lösungsmittels der Rückstand destilliert. Man erhält 6 g 3-Trifluormethoxy-4,6-difluorbenzylalkohol vom Kp = 97–99°C/15 mm.

#### 3-Trifluormethoxy-2-fluor-benzylalkohol

In einer Rührapparatur werden 4 g Natriumborhydrid in 75 ml Dioxan suspendiert und bei 20°C innerhalb von 75 Minuten 14,5 g 3-Trifluormethoxy-2-fluor-benzoylfluorid gelöst in 30 ml trockenem Dioxan zugetropft. Dann werden 2 Stunden bei 20°C und 1 Stunde bei 100°C nachgerührt, abgekühlt und nacheinander 30 ml Wasser und 70 ml 20%ige Schwefelsäure zugetropft. Die Lösung wird mehrmals mit Methylenchlorid extrahiert, das Lösungsmittel wieder abgedampft und das Produkt destilliert. Es werden 8,3 g 3-Trifluormethoxy-2-fluor-benzylalkohol von Kp = 92–93°C/16 mm erhalten.

Analog den beiden vorstehenden Beispielen werden folgende Benzylalkohole erhalten:

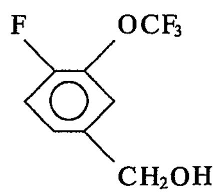

Kp = 94–95°C/15 mm

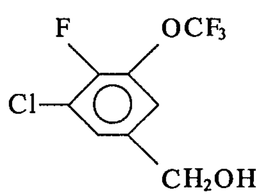

Kp = 101–103°C/16 mm

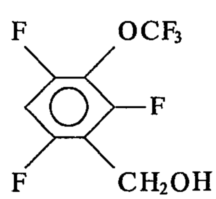

Kp = 90–91°C/13 mm

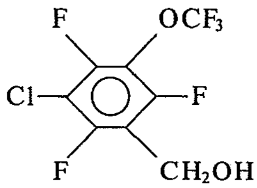

Kp = 98–100°C/16 mm

3-Trifluormethoxy-6-fluorbenzylalkohol

10 g 3-Trifluormethoxy-6-fluorbenzylbromid werden mit 24,6 g 50%iger Kalilauge, 100 ml Toluol, 1 g Tetrabutylammoniumbromid und 4,5 g Essigsäure vermischt und 2 Stunden zum Sieden erhitzt. Nach dem Abkühlen trennt man die Toluolphase ab, wäscht mit Wasser und destilliert das Toluol im Vakuum ab. Der zurückbleibende Benzylalkohol hat eine Reinheit von 93%. Brechungsindex $n_D^{20} = 1{,}441$.

Analog werden erhalten:

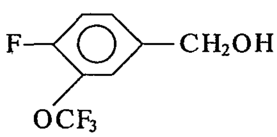

Kp = 98–100°C/17 mm

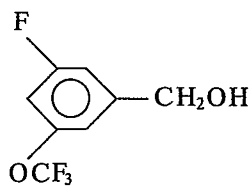

Kp = 104°C/17 mm

3-Trifluormethoxy-4-fluro-α-cyano-benzylalkohol

7,2 g Kaliumcyanid werden unter Kühlung in 30 ml Wasser und 6 ml Ethanol gelöst. Dann wird unter Kühlung bei 0–5°C 19,35 g 3-Trifluormethoxy-4-fluor-benzaldehyd zugegeben. Nach 20 Minuten Nachrühren wird zwischen 0 und 10°C eine Mischung aus 7 ml konzentrierter Schwefelsäure und 18 ml Wasser zugetropft. Man rührt noch 2 Stunden nach und läßt dabei auf Raumtemperatur kommen. Nach zweimaligem Extrahieren mit Methylenchlorid wird die organische Phase getrocknet und das Lösungsmittel abdestilliert. Man erhält 3-Trifluormethoxy-4-fluor-α-cyano-benzylalkohol als farbloses Öl.

Die Struktur wird durch IR- und NMR-Spektren bewiesen.

B. Herstellung der Benzylhalogenide (V)

3-Trifluormethoxy-4-fluor-benzylbromid

Man erhitzt 30 g 3-Trifluormethoxy-4-fluor-toluol mit 30 g N-Bromsuccinimid (NBS) in 200 ml Tetrachlorkohlenstoff und einer Spatelspitze Azo-bis-diisobutyronitril so lange zum Sieden, bis das gesamte Festprodukt oben schwimmt. Dann kühlt man auf 20°C ab, filtriert und destilliert die Tetrachlorkohlenstofflösung. Bei Kp = 85–87°C/14 mm gehen 22 g 3-Trifluormethoxy-4-fluor-benzylbromid über.

Analog werden erhalten:

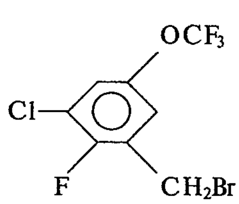

3-Trifluormethoxy-5-chlor-6-fluor-benzylbromid
Kp = 100–104°C/14 mm

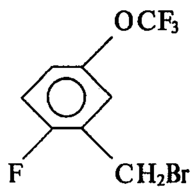

3-Trifluormethoxy-6-fluor-benzylbromid
Kp = 85°C/15 mm
$n_D^{20}$ = 1,4655

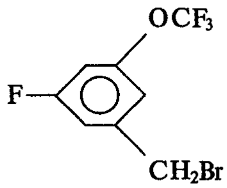

3-Trifluormethoxy-5-fluor-benzylbromid
Kp = 90–92°C/15 mm

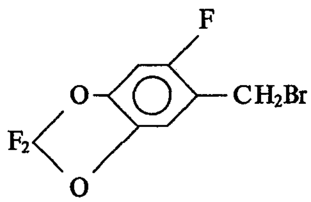

Difluor-3,4-dioxymethylen-6-fluor-benzylbromid
Kp = 102–104°C/15 mm

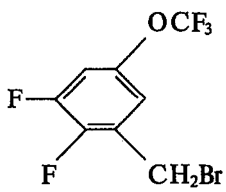

(5,6-Difluor-3-trifluormethoxy-benzylbromid)

Eine Mischung aus 14 g 5,6-Difluor-3-trifluormethoxytoluol, 80 ml Tetrachlorkohlenstoff, 13 g N-Bromsuccinimid und einer Spatelspitze Porophor N werden unter Rückfluß erhitzt, bis das gesamte unlösliche Festprodukt oben schwimmt. Nach Erkalten wird filtriert und das Filtrat destilliert. Bei Kp: 88–90°C/22 mbar gehen 10 g 5,6-Difluor-4-trifluormethoxybenzylbromid über ($n_D^{20}$: 1,4645).

C. Herstellung der Benzoylhalogenide (VI)

3-Trifluormethoxy-4,6-difluor-benzoylfluorid

In einer Chlorierungsapparatur mit UV-Bestrahlung werden 250 g 3-Methoxy-4,6-dichlor-benzoylchlorid vorgelegt und auf 140°C erhitzt. Dann wird gleichzeitig Chlor eingeleitet und eine Lösung von 2 g Benzoylperoxid in Chloroform innerhalb von ca. 3 Stunden zugetropft. Die Reaktion wird mittels GC-Analyse verfolgt und abgebrochen, wenn kein weiterer Umsatz zum gewünschten Chlorierungsprodukt mehr erfolgt. Durch Destillation werden 280 g 3-Trichlormethoxy-4,6-dichlor-benzoylchlorid vom Kp = 132–134°C/0,6 mm erhalten.

Man suspendiert 90 g trockenes Kaliumfluorid in 200 ml wasserfreiem Tetramethylensulfon und fügt 60 g 3-Trichlormethoxy-4,6-dichlor-benzoylchlorid hinzu. Diese Mischung rührt man unter Feuchtigkeitsausschluß für 5 Stunden bei 200–210°C Innentemperatur. Dann destilliert man das Produkt über eine kleine Kolonne mit Rücklaufteiler. Man erhält 21 g 3-Trifluormethoxy-4,6-difluor-benzoylfluorid vom Kp = 56–59°C/13 mm.

Herstellung von 3-Methoxy-4,6-dichlor-benzoylchlorid

In einer Rührapparatur werden 140 g 3-Methoxy-4,6-dichlorbenzoesäure in 280 ml Chlorbenzol vorgelegt und bei 55–60°C so lange Thionylchlorid zugetropft, bis eine klare Lösung entstanden ist und keine Gasentwicklung mehr erfolgt. Durch fraktionierte Destillation werden 108 g 3-Methoxy-4,6-dichlorbenzoylchlorid (Kp = 126–128°C/0,5 mm) erhalten.

3-Trifluormethoxy-2-fluor-benzoylfluorid

In einer Rührapparatur legt man 85 g wasserfreies Kaliumfluorid in 185 ml trockenem Tetramethylensulfon vor und fügt 58 g 3-Trichlormethoxy-2-chlor-benzoylchlorid hinzu. Dann rührt man unter

Feuchtigkeitsausschluß für 4 Stunden bei 200—210°C, kühlt ab und destilliert über eine kleine Kolonne mit Rückflußteiler. Man erhält 14,5 g 3-Trifluormethoxy-2-fluorbenzoylfluorid vom Kp = 57—60°C/ 20 mm.

3-Trichlormethoxy-2-chlor-benzoylchlorid

In einer Chlorierungsapparatur werden 180 g 3-Methoxy-2-chlor-benzoylchlorid bei 140°C unter UV-Bestrahlung vorgelegt und gleichzeitig Chlor eingeleitet und eine Lösung von Benzoylperoxid in Chloroform während der gesamten Chlorierungszeit zugetropft. Zeigt die GC-Analyse keine niederchlorierten Verbindungen mehr, wird der Ansatz aufdestilliert. Man erhält 174 g 3-Trichlormethoxy-2-chlor-benzoylchlorid vom Kp = 130—131°C/1 mm, $n_D^{20} = 1,5745$.

3-Trifluormethoxy-2,4,6-trifluor-benzoylfluorid

wird analog obigen Beispielen durch Fluorierung mit KF in Tetramethylensulfon aus 3-Trifluormethoxy-2,4,6-trichlorbenzoylchlorid erhalten. Ausbeute 52% der Theorie; Kp = 90—92°C/13 mm.

Unter diesen Bedingungen wurden außerdem erhalten:

3-Trifluormethoxy-4-fluor-benzoylfluorid aus 3-Trifluormethoxy-4-chlor-benzoylfluorid

3-Trifluormethoxy-4-fluor-5-chlor-benzoylfluorid aus 3-Trifluormethoxy-4,5-dichlor-benzoylfluorid

Die Ausgangsverbindungen mit einem oder mehreren Chloratomen im Phenylkern können durch Chlorierung von 3-Trifluormethoxy-benzoylfluorid erhalten werden. Die Chlorierung wird durch GC und/oder durch Messung des Brechungsindexes verfolgt und je nach gewünschtem Produkt zum optimalen Zeitpunkt abgebrochen.

In einer Rührapparatur mit Chloreinleitung legt man 200 g 3-Trifluormethoxy-benzoylfluorid und 5 g gepulvertes Eisensulfid bei Rückfluß vor (ca. 175—178°C) und leitet Chlor ein. In dem Maße wie der Siedepunkt des Chlorierungsgemisches steigt, erhöht man die Reaktionstemperatur, so daß ständig unter Rückfluß chloriert wird. Wenn die Reaktionslösung einen Brechungsindex von $n_D^{20} = 1,5135$ erreicht hat, wird mit Stickstoff ausgeblasen und destilliert. Durch Fraktionierung über eine Kolonne erhält man 65 g 3-Trifluormethoxy-2,4,6-trichlorbenzoylfluorid (Kp = 115—118°C/14 mm) und 56 g 3-Trifluormethoxy-tetrachlor-benzoylfluorid (Kp = 126—128°C/14 mm).

3-Trifluormethoxy-2,4,6-trifluor-5-chlor-benzoylfluorid

wird analog den oben angegebenen Beispielen aus 3-Trifluormethoxy-tetrachlorbenzoylfluorid durch Fluorierung mit KF in Tetramethylen-sulfon erhalten. Kp = 103—104°C/14 mm. Aus 36 g Tetrachlorverbindung werden 18 g 3-Trifluormethoxy-2,4,6-trifluor-5-chlor-benzoylfluorid erhalten.

D. Herstellung von 3-Trifluormethoxy-4-fluor-benzaldehyd

3-Trifluormethoxy-4-fluor-benzoylfluorid wird mit 1n-Natronlauge zur Säure verseift, die durch Ansäuern in Freiheit gesetzt wird und mit Thionylchlorid in das 3-Trifluormethoxy-4-fluor-benzoylchlorid überführt wird. 24,2 g 3-Trifluormethoxy-4-fluorbenzoylchlorid werden langsam bei Raumtemperatur zu 12,4 g Trimethylphosphit getropft. Nach beendetem Zutropfen wird noch 1 Stunde auf 100°C erhitzt. Das rohe 3-Trifluormethoxy-4-fluor-benzoyl-dimethyl-phosphonat wird in 100 ml 1,2-Dimethoxyethan gelöst und mit 1,2 g Natriumborhydrid in 30 ml Wasser versetzt. Nach beendetem Zutropfen wird sofort auf einen pH-Wert von ca. 5 eingestellt, mit Wasser verdünnt und mit Methylenchlorid extrahiert. Diese Methylenchloridlösung wird 3—4 Stunden mit 100 ml 1n NaOH bei Raumtemperatur gerührt. Dann trennt man die $CH_2Cl_2$-Phase ab, wäscht neutral und trocknet mit Natriumsulfat.

Man erhält durch Destillation im Vakuum 8,8 g 3-Trifluormethoxy-4-fluor-benzaldehyd vom Kp = 82—84°C/15 mm.

E. Herstellung der Toluole (VII)

3-Trifluormethoxy-4-fluor-toluol

In einem VA-Reaktionsgefäß legt man bei 0°C 500 g wasserfreie Flußsäure vor und gibt dann eine Mischung aus 500 ml $CCl_4$ und 100 g 2-Fluor-5-methyl-phenol zu. Unter Eigendruck wird für 8 Stunden auf 140°C erhitzt. Nach Abkühlen auf 20°C destilliert man den Ansatz über eine kleine Kolonne und erhält nach einem Vorlauf 40 g 3-Trifluormethoxy-4-fluor-toluol vom Kp = 137°C; $n_D^{20} = 1,4092$.

3-Trifluormethoxy-5-chlor-6-fluor-toluol

In eine Mischung aus 43 g 3-Trifluormethoxy-6-fluor-toluol und 0,5 g Jod in 70 ml Tetrachlorkohlenstoff wird bis zur Sättigung Chlor eingeleitet. Die Temperatur steigt anfangs exotherm auf 35°C und wird dann bis Ende der Chloraufnahme auf 80°C gesteigert. Die abgekühlte Lösung wird über eine kleine Kolonne mit Wilsonspirale destilliert. Man erhält beim Kp=64–66°C/18 mm 18 g 3-Trifluormethoxy-5-chlor-6-fluor-toluol.

3-Trifluormethoxy-6-fluor-toluol

In 300 ml Methylenchlorid werden 85 g Nitrosyltetrafluoroborat suspendiert. Bei 5°C tropft man 130 g 4-Trifluormethoxy-2-methyl-anilin gelöst in 450 ml $CH_2Cl_2$ in ca. 3 Stunden zu. Nachdem man noch 30 Minuten bei 20°C nachgerührt hat, saugt man das Fettprodukt ab und wäscht mit etwas kaltem Petrolether nach. Das getrocknete Produkt wird in einer Destillationsapparatur vorgelegt und langsam erhitzt. Bei einer Badtemperatur von 120–140°C setzt Gasentwicklung ein und das 3-Trifluormethoxy-6-fluor-toluol beginnt zu destillieren. Gegen Ende der Reaktion erhitzt man auf 180–200°C und bläst letzte Reste an Produkt über. Durch erneute Destillation erhält man 108 g 3-Trifluormethoxy-6-fluortoluol vom Kp = 120–122°C; $n_D^{20}$ = 1,4052.

Analog vorstehendem Beispiel werden erhalten:

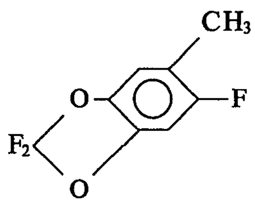

Difluor-3,4-dioxymethylen-6-fluor-toluol
Kp = 65°C/15 mm

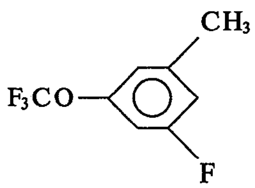

3-Trifluormethoxy-5-fluor-toluol
Kp = 38–42°C/17 mm

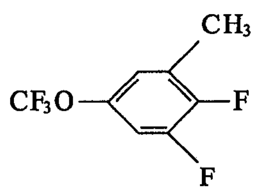

5,6-Difluor-3-trifluormethoxy-toluol

in 150 ml Methylenchlorid werden bei 5°C 41 g Nitrosyltetrafluorborat suspendiert und 65 g 5-Amino-6-fluor-3-trifluormethoxy-toluol gelöst in 225 ml Methylenchlorid zugetropft. Nachdem die Reaktionsmischung für 90 Minuten bei 0°C und 30 Minuten bei 20°C gerührt wurde, wird der Niederschlag abgesaugt. Das Festprodukt hat einen Rohschmelzpunkt on 114–126°C und wird ohne Reinigung in einer Destillationsapparatur erhitzt. Bei ca. 170°C beginnt die Zersetzung unter Entwicklung von Bortrifluorid und Destillation des Produktes. Die Reaktion wird bei 190°C vervollständigt. Redestillation liefert bei Kp: 110°C 14 g 5,6-Difluor-3-trifluormethoxytoluol.

F. Herstellung der Aniline (IX)

4-Trifluormethoxy-2-methyl-anilin

In einer VA-Apparatur werden bei 0°C eine Mischung aus 850 ml wasserfreier Flußsäure, 250 g 4-Amino-3-methyl-phenol und 800 ml Tetrachlorkohlenstoff vorgelegt. Diese Mischung wird für 12 Stunden auf 140°C erhitzt. Nach dem Abkühlen wird der Ansatz auf Wasser ausgetragen und mit Natronlauge alkalich gestellt. Dann wird diese Mischung in einer Destillationsapparatur auf 100°C erhitzt und Wasserdampf durchgeblasen bis keine organische Phase im Destillat mehr erkennbar ist.

Dann wird die organische Phase aus dem Destillat im Scheidetrichter abgetrennt und über Natriumsulfat getrocknet. Man erhält 158 g 4-Trifluormethoxy-2-methyl-anilin vom Kp = 90–92°C/15 mm.

Analog wurde erhalten:

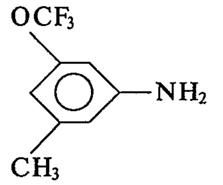

3-Trifluormethoxy-5-methyl-anilin
Kp = 102°C/16 mm

Herstellung von Difluor-3,4-dioxymethylen-6-methyl-anilin

Zu einer Nitriersäure, hergestellt aus 35 ml Salpetersäure (D = 1,41) und 40 ml Schwefelsäure (D = 1,84), wurden bei 0–5°C 34,4 g (0,2 Mol) Difluor-3,4-dioxymethylen-toluol (hergestellt nach DT-OS 2 819 788) getropft. Man rührt 1 Stunde bei 10°C, dann eine weitere Stunde bei 20°C und erwärmt zum Schluß für 5 Minuten auf 40°C. Der abgekühlte Ansatz wird auf 100 g Eis gegossen und mit Methylenchlorid extrahiert. Die organische Phase wird neutral gewaschen, getrocknet und nach Abdestillation des Lösungsmittels im Vakuum destilliert. Man erhält 36 g Difluor-3,4-dioxy-methylen-6-nitro-toluol vom Kp = 58–62°C/0,3 mm. Das Produkt wird in 120 ml Ethanol gelöst und in einem Hydrierautoklaven mit 10 g Raney-Nickel versetzt. Es wird bei 45°C und 50 bar/$H_2$ hydriert. Nach Abkühlen und Entspannen wird der Katalysator abfiltriert und das Filtrat destilliert. Man erhält 22 g Difluor-3,4-dioxy-methylen-6-methyl-anilin vom Kp = 108–112°C/15 mm.

5-Amino-6-fluor-trifluormethoxy-toluol

In einer Hydrierapparatur werden 80 g 5-Nitro-6-fluor-3-trifluor-methoxy-toluol gelöst in 350 ml Methanol in Gegenwart von 5 g Raney-Nickel bei 30–35°C mit 30 bar Wasserstoff bei Sättigung hydriert. Anschließend wird der Katalysator abfiltriert und das Lösungsmittel abdestilliert. Bei Kp: 73–77°C/20 mbar destillieren dann 65 g 5-Amino-6-fluor-3-trifluor-methoxy-toluol ($n_D^{20}$: 1,4538).

5-Nitro-6-fluor-3-trifluormethoxy-toluol

100 g 6-Fluor-3-trifluormethoxy-toluol werden bei 50°C vorgelegt und eine Mischung aus 140 ml Salpetersäure (D: 1,41) und 170 ml konzentrierte Schwefelsäure zugetropft. Nach Ende der Zugabe wird für eine Stunde bei 10°C, eine Stunde bei 20°C und 15 Minuten bei 40°C gerührt und anschließend auf Eis gegossen. die organische Phase wird abgetrennt, getrocknet und destilliert. Nach einem Vorlauf von Ausgangsmaterial gehen bei Kp: 98–100°C/18 mbar 90 g 5-Nitro-6-fluor-3-trifluormethoxy-toluol ($n_D^{20}$: 1,4565) über.

Beispiel A

$LD_{100}$-Test

| | |
|---|---|
| Testtiere: | Sitophilus granarius |
| Zahl der Testtiere: | 25 |
| Lösungsmittel: | Aceton |

2 Gewichtsteile Wirkstoff werden in 1000 Volumenteilen Lösungsmittel aufgenommen. Die so erhaltene Lösung wird mit weiterem Lösungsmittel auf die gewünschten Konzentrationen verdünnt.

2,5 ml Wirkstofflösung werden in eine Petrischale pipettiert. Auf dem Boden der Petrischale befindet sich ein Filterpapier mit einem Durchmesser von etwa 9,5 cm. Die Petrischale bleibt so lange offen stehen, bis das Lösungsmittel vollständig verdunstet ist. Je nach Konzentration der Wirkstofflösung ist die Menge Wirkstoff pro $m^2$ Filterpapier verschieden hoch. Anschließend gibt man die angegebene Anzahl der Testtiere in die Petrischale und bedeckt sie mit einem Glasdeckel.

Der Zustand der Testtiere wird 3 Tage nach Ansetzen der Versuche kontrolliert. Bestimmt wird die Abtötung in %. Dabei bedeutet 100%, daß alle Testtiere abgetötet wurden; 0% bedeutet, daß keine Testtiere abgetötet wurden.

Bei diesem Test zeigen z. B. die folgenden Verbindungen der Herstellungsbeispiele überlegene Wirkung gegenüber dem Stand der Technik: 1, 4, 2, 22, 14, 20, 7.

## Beispiel B

### Grenzkonzentrations-Test/Bodeninsekten

| | | |
|---|---|---|
| Testinsekt: | Agrotis segetum-Larven (im Boden) | |
| Lösungsmittel: | 3 Gewichtsteile | Aceton |
| Emulgator: | 1 Gewichtsteil | Alkylarylpolyglykoläther |

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit dem Boden vermischt. Dabei spielt die Konzentration des Wirkstoffs in der Zubereitung praktisch keine Rolle, entscheidend ist allein die Wirkstoffgewichtsmenge pro Volumeneinheit Boden, welche in ppm (= mg/l) angegeben wird. Man füllt den Boden in Töpfe und läßt diese bei Raumtemperatur stehen.

Nach 24 Stunden werden die Testtiere in den behandelten Boden gegeben und nach weiteren 2 bis 7 Tagen wird der Wirkungsgrad des Wirkstoffs durch Auszählen der toten und lebenden Testinsekten in % bestimmt. Der Wirkungsgrad ist 100%, wenn alle Testinsekten abgetötet worden sind, er ist 0%, wenn noch genau so viele Testinsekten leben wie bei der unbehandelten Kontrolle.

Bei diesem Test zeigen z. B. die folgenden Verbindungen der Herstellungsbeispiele überlegene Wirkung gegenüber dem Stand der Technik: 1,4.

## Beispiel C

### $LT_{100}$-Test für Dipteren

| | |
|---|---|
| Testtiere: | Aedes aegypti |
| Zahl der Testtiere: | 25 |
| Lösungsmittel: | Aceton |

2 Gewichtsteile Wirkstoff werden in 1000 Volumenteilen Lösungsmittel aufgenommen. Die so erhaltene Lösung wird mit weiterem Lösungsmittel auf die gewünschten geringeren Konzentrationen verdünnt.

2,5 ml Wirkstofflösung werden in eine Petrischale pipettiert. Auf dem Boden der Petrischale befindet sich ein Filterpapier mit einem Durchmesser von etwa 9,5 cm. Die Petrischale bleibt so lange offen stehen, bis das Lösungsmittel vollständig verdunstet ist. Je nach Konzentration der Wirkstofflösung ist die Menge Wirkstoff pro $m^2$ Filterpapier verschieden hoch. Anschließend gibt man die angegebene Anzahl der Testtiere in die Petrischale und bedeckt sie mit einem Glasdeckel.

Der Zustand der Testtiere wird laufend kontrolliert.Es wird diejenige Zeit ermittelt, welche für einen 100%igen knock down-Effekt notwendig ist.

Bei diesem Test zeigen z. B. die folgenden Verbindungen der Herstellungsbeispiele überlegene Wirkung gegenüber dem Stand der Technik: 1, 4, 10, 13, 22, 11, 15, 2, 14, 20, 9, 22, 6, 7, 5, 23, 24.

## Beispiel D

### Drosophila-Test

| | | |
|---|---|---|
| Lösungsmittel: | 3 Gewichtsteile | Aceton |
| Emulgator: | 1 Gewichtsteil | Alkylarylpolyglykoläther |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

1 $cm^3$ der Wirkstoffzubereitung wird auf eine Filterpapierscheibe (7 cm Durchmesser) aufpipettiert. Man legt diese naß auf die Öffnung eines Glasgefäßes, in dem sich 50 Taufliegen (Drosophila melanogaster) befinden und bedeckt es mit einer Glasplatte.

Nach der gewünschten Zeit bestimmt man die Abtötung in %. Dabei bedeutet 100%, daß alle Fliegen abgetötet wurden; 0% bedeutet, daß keine Fliegen abgetötet wurden.

Bei diesem Test zeigen z. B. die folgenden Verbindungen der Herstellungsbeispiele überlegene Wirksamkeit gegenüber dem Stand der Technik: 5, 11, 10, 13, 15, 12, 9, 1, 3, 2, 20, 14, 4, 22, 6, 25, 7, 23, 24.

Beispiel E

Tetranychus-Test (resistent)

| | | |
|---|---|---|
| Lösungsmittel: | 3 Gewichtsteile | Aceton |
| Emulgator: | 1 Gewichtsteil | Alkylarylpolyglykoläther |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Bohnenpflanzen (Phaseolus vulgaris), die stark von alen Entwicklungsstadien der gemeinen Spinnmilbe oder Bohnenspinnmilbe (Tetranychus urticae) befallen sind, werden durch Tauchen in die Wirkstoffzubereitung der gewünschten Konzentration behandelt.

Nach der gewünschten Zeit wird die Abtötung in % bestimmt. Dabei bedeutet 100%, daß alle Spinnmilben abgetötet wurden; 0% bedeutet, daß keine Spinnmilben abgetötet wurden.

Bei diesem Test zeigen z. B. die folgenden Verbindungen der Herstellungsbeispiele überlegene Wirksamkeit gegenüber dem Stand der Technik: 1, 4.

Beispiel F

Test mit Boophilus microplus resistent

Lösungsmittel: 35 Gewichtsteile Äthylenglykolmonomethyläther
35 Gewichtsteile Nonylphenolpolyglykoläther

Zur Herstellung einer zweckmäßigen Wirkstoffzubereitung vermischt man drei Gewichtsteile Wirkstoff mit sieben Gewichtsteilen des oben angegebenen Lösungsmittel-Gemisches und verdünnt das so erhaltene Konzentrat mit Wasser auf die gewünschte Konzentration.

10 adulte Boophilus microplus res. werden in die zu testende Wirkstoffzubereitung 1 Minute getaucht. Nach Überführung in Plastikbecher und Aufbewahrung in einem klimatisierten Raum wird der Abtötungsgrad bestimmt.

Bei diesem Test zeigen z. B. die folgenden Verbindungen der Herstellungsbeispiele überlegene Wirkung gegenüber dem Stand der Technik: 2, 7, 22.

Beispiel G

Test mit Stomoxys calcitrans

Lösungsmittel: 35 Gewichtsteile Äthylenglykolmonomethyläther
35 Gewichtsteile Nonylphenolpolyglykoläther

Zur Herstellung einer zweckmäßigen Wirkstoffzubereitung vermischt man drei Gewichtsteile Wirkstoff mit sieben Gewichtsteilen des oben angegebenen Lösungsmittelgemisches und verdünnt das so erhaltene Konzentrat mit Wasser auf die jeweils gewünschte Konzentration.

10 adulte Stomoxys calcitrans werden in Petrischalen gebracht, die Filterpapierscheiben entsprechender Größe enthalten, die einen Tag vor Versuchsbeginn mit 1 ml der zu testenden Wirkstoffzubereitung durchtränkt wurden. Nach 3 Stunden wird der Abtötungsgrad bestimmt.

Bei diesem Test zeigen z. B. die folgenden Verbindungen der Herstellungsbeispiele überlegene Wirkung gegenüber dem Stand der Technik: 7, 9, 10.

Beispiel G

Test mit Musca autumnalis

Lösungsmittel: 35 Gewichtsteile Äthylenglykolmonomethyläther
35 Gewichtsteile Nonylphenolpolyglykoläther

Zur Herstellung einer zweckmäßigen Wirkstoffzubereitung vermischt man drei Gewichtsteile Wirkstoff mit sieben Gewichtsteilen des oben angegebenen Lösungsmittelgemisches und verdünnt das so erhaltene Konzentrat mit Wasser auf die jeweils gewünschte Konzentration.

10 adulte Musca autumnalis werden in Petrischalen gebracht, die Filterpapierscheiben entsprechender Größe enthalten, die einen Tag vor Versuchsbeginn mit 1 ml der zu testenden Wirkstoffzubereitung

durchtränkt wurden. Nach 3 Stunden wird der Abtötungsgrad bestimmt.

Bei diesem Test zeigen z. B. die folgenden Verbindunen der Herstellungsbeispiele überlegene Wirkung gegenüber dem Stand der Technik: 7, 9, 22, 10.

Beispiel H

Test mit Lucilia cuprina res.-Larven

Emulgator: 35 Gewichtsteile Äthylenglykolmonomethyläther
35 Gewichtsteile Nonylphenolpolyglykoläther

Zur Herstellung einer zweckmäßigen Wirkstoffzubereitung vermischt man drei Gewichtsteile Wirkstoff mit sieben Gewichtsteilen des oben angegebenen Lösungsmittel-Gemisches und verdünnt das so erhaltene Konzentrat mit Wasser auf die jeweils gewünschte Konzentration.

Etwa 20 Lucilia cuprina res.-Larven werden in ein Teströhrchen gebracht, welches ca. 1 $cm^2$ Pferdefleisch und 0,5 ml der Wirkstoffzubereitung enthält. Nach 24 Stunden wird der Abtötungsgrad bestimmt.

Bei diesem Test zeigen z. B. die folgenden Verbindungen der Herstellungsbeispiele überlegene Wirkung gegenüber dem Stand der Technik: 2, 4, 7, 22.

**Patentansprüche**

1. Benzylester der Formel (I)

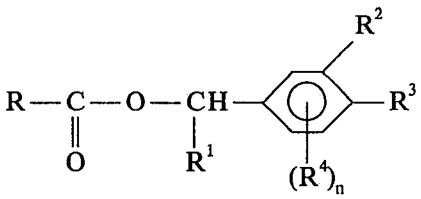

(I)

in welcher

R für den Rest einer bei Pyrethroiden verwendbaren Säure steht,
$R^1$ für Wasserstoff oder Cyan steht,
$R^2$ für Trifluormethoxy steht und
$R^3$ für Wasserstoff oder Halogen steht oder
$R^2$ und $R^3$ gemeinsam für fluorsubstituiertes Dioxymethylen stehen,
$R^4$ für Halogen steht und
n für 0, 1, 2 oder 3 steht,

mit der Maßgabe, daß für den Fall, daß $R^2$ für $OCF_3$ steht, einer der Reste $R^3$ oder $R^4$ für Fluor stehen muß und ferner mit der Maßgabe, daß wenn $R^2$ und $R^3$ für fluorsubstituiertes Dioxymethylen stehen, n für 1 steht und $R^4$ in 6-Stellung für Fluor stehen muß.

2. Verfahren zur Herstellung der Benzylester der Formel (I)

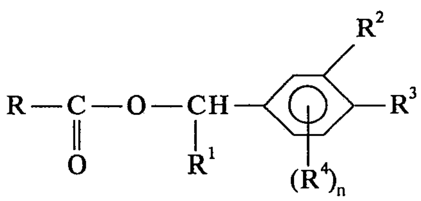

(I)

in welcher

R für den Rest einer bei Pyrethroiden verwendbaren Säure steht,
$R^1$ für Wasserstoff oder Cyan steht,
$R^2$ für Trifluormethoxy steht und
$R^3$ für Wasserstoff oder Halogen steht oder
$R^2$ und $R^3$ gemeinsam für fluorsubstituiertes Dioxymethylen stehen,
$R^4$ für Halogen steht und
n für 0, 1, 2 oder 3 steht,

mit der Maßgabe, daß für den Fall, daß $R^2$ für $OCF_3$ steht, einer der Reste $R^3$ oder $R^4$ für Fluor stehen muß und ferner mit der Maßgabe, daß wenn $R^2$ und $R^3$ für fluorsubstituiertes Dioxymethylen stehen, n für 1 steht und $R^4$ in 6-Stellung für Fluor stehen muß, dadurch gekennzeichnet, daß man eine bei Pyrethroiden verwendbare Säure oder ein reaktionsfähiges Derivat derselben der Formel (II)

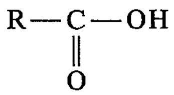
(II)

in welcher
R die oben angegebene Bedeutung besitzt,
mit einem Alkohol oder einem reaktionsfähigen Derivat desselben der Formel (III)

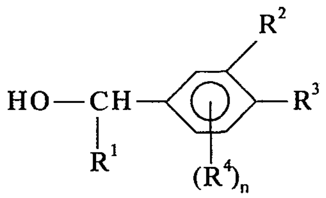
(III)

in welcher
$R^1$–$R^4$ und n die oben angegebene Bedeutung besitzen,
umsetzt.

3. Benzylalkohole der Formel (III)

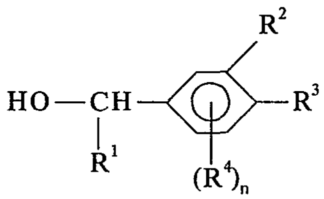
(III)

in welcher

$R^1$ für Wasserstoff oder Cyan steht,
$R^2$ für Trifluormethoxy steht und
$R^3$ für Wasserstoff oder Halogen steht oder
$R^2$ und $R^3$ gemeinsam für fluorsubstituiertes Dioxymethylen stehen,
$R^4$ für Halogen steht und
n für 0, 1, 2 oder 3 steht,

mit der Maßgabe, daß für den Fall, daß $R^2$ für $OCF_3$ steht, einer der Reste $R^3$ oder $R^4$ für Fluor stehen muß und ferner mit der Maßgabe, daß wenn $R^2$ und $R^3$ für fluorsubstituiertes Dioxymethylen stehen, n für 1 steht und $R^4$ in 6-Stellung für Fluor stehen muß.

4. Verfahren zur Herstellung der Benzylalkohole der Formel (III) gemäß Anspruch 3, dadurch gekennzeichnet, daß man

a) Aldehyde der Formel (IV)

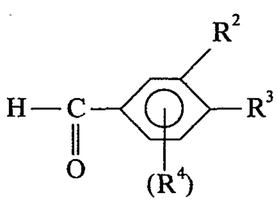
(IV)

in welcher
$R^2$–$R^4$ und n die in Anspruch 3 angegebene Bedeutung besitzen,

$a_1$) für den Fall, daß $R^1$ für Wasserstoff steht, reduziert oder
$a_2$) für den Fall, daß $R^1$ für Cyan steht, mit Cyaniden (gegebenenfalls in situ) zum Cyanhydrin umsetzt, oder

b) Benzylhalogenide der Formel (V)

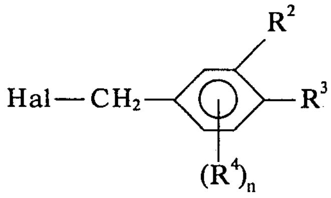

(V)

in welcher $R^2$–$R^4$ und n die in Anspruch 3 angegebene Bedeutung haben und Hal für Chlor oder Brom steht, hydrolysiert oder

c) Säurehalogenide der Formel (VI)

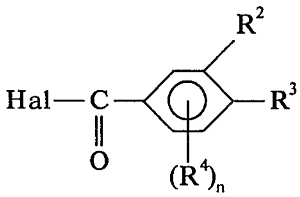

(VI)

in welcher $R^2$–$R^4$ und n die in Anspruch 3 angegebene Bedeutung haben und Hal für Fluor, Chlor oder Brom steht, mit einem Alkali-Boranat reduziert.

5. Aldehyde der Formel (IV)

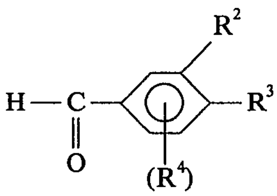

(IV)

in welcher $R^2$–$R^4$ und n die oben angegebene Bedeutung besitzen.

6. Verfahren zur Herstellung der Aldehyde der Formel (IV) gemäß 5. (oben), dadurch gekennzeichnet, daß man

a) Benzylhalogenide der Formel (V) mit Urotropin umsetzt und anschließend hydrolysiert oder

b) Verbindungen der Formel (VI)

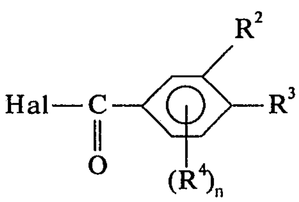

(VI)

in welcher $R^2$–$R^4$ und n die oben angegebene Bedeutung besitzen und Hal für Chlor oder Brom steht, mit Trialkylphosphit umsetzt, anschließend mit Alkaliboranat reduziert und dann hydrolysiert.

7. Benzylester gemäß Anspruch 1 der Formel (XIII)

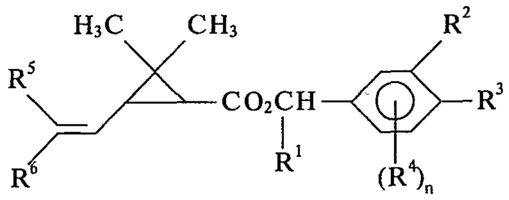

(XIII)

in welcher

$R^1$ für Wasserstoff oder Cyan steht,
$R^2$ für Trifluormethoxy steht,
$R^3$ für Wasserstoff oder Fluor steht oder
$R^2$ und $R^3$ gemeinsam für Difluordioxymethylen stehen,
$R^4$ für Fluor oder Chlor steht und
n für 0, 1, 2, 3 stehen kann, mit der Maßgabe, daß für den Fall, daß $R^2$ für $OCF_3$ steht, einer der Reste $R^3$ oder $R^4$ für Fluor stehen muß und ferner mit der Maßgabe, daß wenn
$R^2$ und $R^3$ für fluorsubstituiertes Dioxymethylen stehen, n für 1 steht und $R^4$ in 6-Stellung für Fluor stehen muß,
$R^5$ und $R^6$ gleich oder verschieden sind und für Methyl, Halogen oder Halogenalkyl mit 1—4 Kohlenstoffatomen stehen.

8. Benzylester gemäß Anspruch 7 der Formel (XIII), in welcher
$R^1$—$R^4$ und n die in Anspruch 7 angegebenen Bedeutungen besitzen,

$R^5$ für Chlor steht und
$R^6$ für Chlor, Trifluormethyl oder Pentafluorethyl steht.

9. Schädlingsbekämpfungsmittel, gekennzeichnet durch einen Gehalt an mindestens einem Benzylester der Formel (I).

10. Verwendung der Benzylester der Formel (I) zur Bekämpfung von Schädlingen.

**Claims**

1. Benzyl esters of the formula (I)

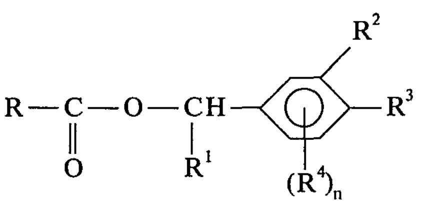

(I)

in which

R represents the radical of an acid which can be used in pyrethroids,
$R^1$ represents hydrogen or cyano,
$R^2$ represents trifluoromethoxy and
$R^3$ represents hydrogen or halogen or
$R^2$ and $R^3$ together represent fluorine-substituted dioxymethylene,
$R^4$ represents halogen and
n represents 0, 1, 2 or 3,

with the proviso that in the case where $R^2$ represents $OCF_3$ one of the radical $R^3$ or $R^4$ must represent fluorine and furthermore with the proviso that when $R^2$ and $R^3$ represent fluorine-substituted dioxymethylene, n represents 1 and $R^4$ must represent fluorine in the 6-position.

2. Process for the preparation of the benzyl esters of the formula (I)

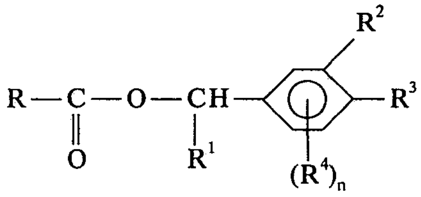

(I)

in which

R represents the radical of an acid which can be used in pyrethroids,
$R^1$ represents hydrogen or cyano,
$R^2$ represents trifluoromethoxy and
$R^3$ represents hydrogen or halogen or
$R^2$ and $R^3$ together represent fluorine-substituted dioxymethylene,
$R^4$ represents halogen and
n represents 0, 1, 2 or 3,

with the proviso that in the case where $R^2$ represents $OCF_3$ one of the radicals $R^3$ or $R^4$ must represent fluorine and furthermore with the proviso that when $R^2$ and $R^3$ represent fluorine-substituted dioxymethylene n represents 1 and $R^4$ must represent fluorine in the 6-position, characterised in that an acid which can be used in pyrethroids or a reactive derivative thereof of the formula (II)

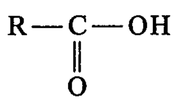

(II)

in which
R has the abovementioned meaning,
is reacted with an alcohol or a reactive derivative thereof of the formula (III)

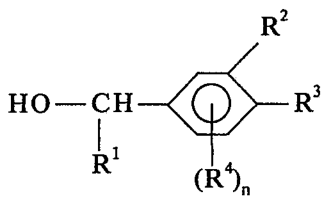

(III)

in which
$R^1$–$R^4$ and n have the abovementioned meaning.

3. Benzyl alcohols of the formula (III)

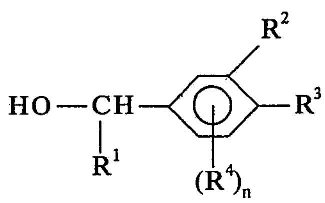

(III)

in which

$R^1$ represents hydrogen or cyano,
$R^2$ represents trifluoromethoxy and
$R^3$ represents hydrogen or halogen or
$R^2$ and $R^3$ together represent fluorine-substituted dioxymethylene,
$R^4$ represents halogen and
n represents 0, 1, 2 or 3,

with the proviso that in the case where $R^2$ represents $OCF_3$ one of the radicals $R^3$ or $R^4$ must represent fluorine and furthermore with the proviso that when $R^2$ and $R^3$ represent fluorine-substituted dioxymethylene n represents 1 and $R^4$ must represent fluorine in the 6-position.

4. Process for the preparation of the benzyl alcohols of the formula (III) according to Claim 3, characterised in that

a) aldehydes of the formula (IV)

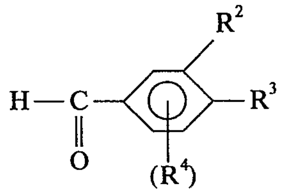

(IV)

in which
$R^2$–$R^4$ and n have the meaning given in Claim 3,

$a_1$) in the case where $R^1$ represents hydrogen, are reduced or
$a_2$) in the case where $R^1$ represents cyano, are reacted with cyanides (if appropriate in situ) to give the cyanohydrin, or

b) benzyl halides of the formula (V)

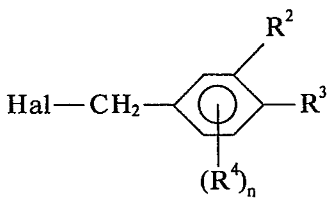

(V)

in which
$R^2$–$R^4$ and n have the meaning given in Claim 3 and
Hal represents chlorine or bromine,
are hydrolysed or

c) acid halides of the formula (VI)

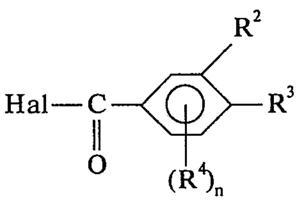

(VI)

in which
$R^2$–$R^4$ and n have the meaning given in Claim 3 and
Hal represents fluorine, chlorine or bromine,
are reduced with an alkali metal boranate.

5. Aldehydes of the formula (IV)

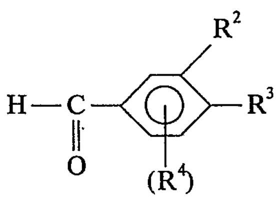

(IV)

in which
$R^2$–$R^4$ and n have the abovementioned meaning.

6. Process for the preparation of the aldehydes of the formula (IV) according to 5 (above), characterised in that

a) benzyl halides of the formula (V) are reacted with Urotropin and then hydrolysed or
b) compounds of the formula (VI)

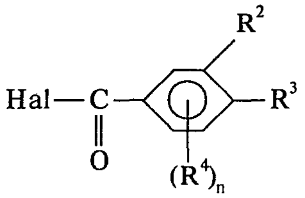

(VI)

in which
$R^2$–$R^4$ and n have the abovementioned meaning and
Hal represents chlorine or bromine,
are reacted with a trialkyl phosphite, then reduced with an alkali metal boranate and then hydrolysed.

7. Benzyl esters according to Claim 1 of the formula (XIII)

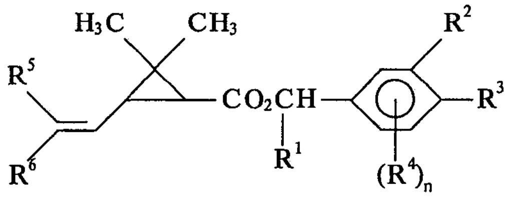

(XIII)

in wich

$R^1$ represents hydrogen or cyano,
$R^2$ represents trifluoromethoxy,
$R^3$ represents hydrogen or fluorine or
$R^2$ and $R^3$ together represents difluorodioxymethylene,
$R^4$ represents fluorine or chlorine and
n can represent 0, 1, 2 or 3 with the proviso that in the case where $R^2$ represents $OCF_3$, one of the radicals $R^3$ or $R^4$ must represent fluorine and furthermore with the proviso that when
$R^2$ and $R^3$ represent fluorine-substituted dioxymethylene n represents 1 and $R^4$ must represent fluorine in the 6-position.
$R^5$ and $R^6$ are identical or different and represent methyl, halogen or halogenoalkyl with 1–4 carbon atoms.

8. Benzyl esters according to Claim 7 of the formula (XIII), in which

$R^1$–$R^4$ and n have the meanings given in Claim 7,
$R^5$ represents chlorine and
$R^6$ represents chlorine, trifluoromethyl or pentafluoroethyl.

9. Agents for combating pests, characterised in that they contain at least one benzyl ester of the formula (I).

10. Use of the benzyl esters of the formula (I) for combating pests.

**Revendications**

1. Esters benzyliques de formule (I)

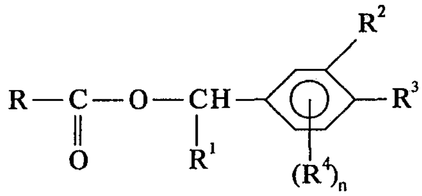

(I)

dans laquelle

R représente le reste d'un acide pouvant être utilisé pour des pyréthroïdes,
$R^1$ est l'hydrogène ou le groupe cyano,
$R^2$ est le groupe trifluorométhoxy et
$R^3$ est l'hydrogène ou un halogène, ou bien
$R^2$ et $R^3$ forment ensemble un groupe dioxyméthylène substitué par du fluor,

$R^4$ est un halogène et
n a la valeur 0, 1, 2 ou 3,

sous réserve que, au cas où $R^2$ représente le groupe $OCF_3$, l'un des restes $R^3$ ou $R^4$ doive représenter du fluor, et sous réserve en outre que lorsque $R^2$ et $R^3$ représentent un groupe dioxyméthylène substitué par du fluor, n possède la valeur 1 et $R^4$ doive représenter du fluor en position 6.

2. Procédé de production des esters benzyliques de formule (I)

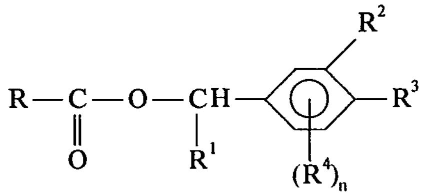

(I)

dans laquelle

R représente le reste d'un acide pouvant être utilisé pour des pyréthroïdes,
$R^1$ est l'hydrogène ou le groupe cyano,
$R^2$ est le groupe trifluorométhoxy et
$R^3$ est l'hydrogène ou un halogène, ou bien
$R^2$ et $R^3$ forment ensemble un groupe dioxyméthylène substitué par du fluor,
$R^4$ est un halogène et
n a la valeur 0, 1, 2 ou 3

sous réserve que, au cas où $R^2$ représente le groupe $OCF_3$, l'un des restes $R^3$ et $R^4$ doive représenter du fluor, et sous réserve en outre que lorsque $R^2$ et $R^3$ représentent un groupe dioxyméthylène substitué par du fluor, n possède la valeur 1 et $R^4$ doive représenter du fluor en position 6, caractérisé en ce qu'on fait réagir un acide pouvant être utilisé pour des pyréthroïdes ou un dérivé réactif d'un tel acide de formule (II)

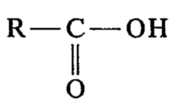

(II)

dans laquelle
R a la définition indiquée ci-dessus,
avec un alcool ou un dérivé réactif d'un alcool de formule (III)

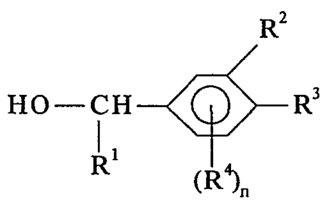

(III)

dans laquelle
$R^1$ à $R^4$ et n ont les définitions données ci-dessus.

3. Alcools benzyliques de formule (III)

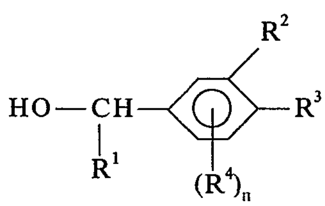

(III)

dans laquelle

$R^1$ est l'hydrogène ou le groupe cyano,
$R^2$ est un groupe trifluorométhoxy et
$R^3$ est l'hydrogène ou un halogène, ou bien
$R^2$ et $R^3$ forment ensemble un groupe dioxyméthylène substitué par du fluor,

$R^4$ est un halogène et
n a la valeur 0, 1, 2 ou 3,

sous réserve que, au cas où $R^2$ représente le groupe $OCF_3$, l'un des restes $R^3$ ou $R^4$ doive représenter du fluor et sous réserve en outre que lorsque $R^2$ et $R^3$ représentent un groupe dioxyméthylène substitué par du fluor, n possède la valeur 1 et $R^4$ doive représenter du fluor en position 6.

4. Procédé de production des alcools benzyliques de formule (III) suivant la revendication 3, caractérisé en ce que,

a) des aldéhydes de formule (IV)

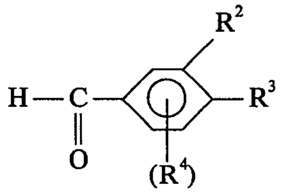

(IV)

dans laquelle
$R^2$—$R^4$ et n ont la définition donnée dans la revendication 3,

$a_1$) sont réduits au cas ou $R^1$ représente l'hydrogène ou bien
$a_2$) sont transformés en cyanhydrine avec des cyanures (éventuellement in situ) pour le cas où $R^1$ représente le groupe cyano, ou bien

b) des halogénures de benzyle de formule (V)

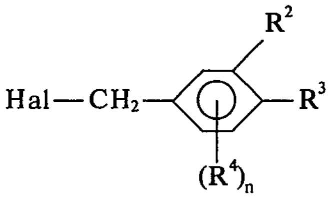

(V)

dans laquelle
$R^2$—$R^4$ et n ont la définition indiquée dans la revendication 3 et
Hal représente le chlore ou le brome,
sont hydrolysés ou bien

c) des halogénures d'acides de formule (VI)

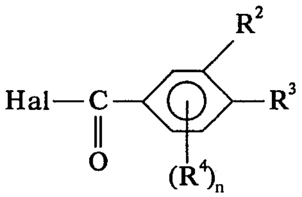

(VI)

dans laquelle
$R^2$—$R^4$ et n ont la définition indiquée dans la revendication 3 et
Hal représente le fluor, le chlore ou le brome,
sont réduits avec un boranate alcalin.

5. Aldéhydes de formule (IV)

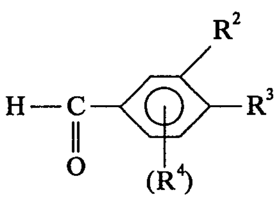

(IV)

dans laquelle

$R^2$–$R^4$ et n ont la définition indiquée ci-dessus.

6. Procédé de production des aldéhydes de formule (IV) suivant 5 (ci-dessus), caractérisé en ce que

a) on fait réagir des halogénures de benzyle de formule (V) avec l'urotropine puis on les hydrolyse, ou bien

b) on fait réagir des composés de formule (VI)

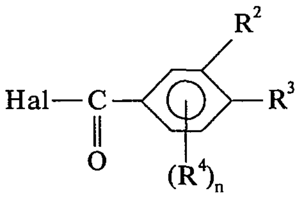

(VI)

dans laquelle
$R^2$–$R^4$ et n ont la définition indiquée ci-dessus et
Hal représente le chlore ou le brome,
avec un phosphite de trialkyle, puis on les reduit avec un boranate alcalin et on les hydrolyse ensuite.

7. Esters benzyliques suivant la revendication 1, de formule (XIII)

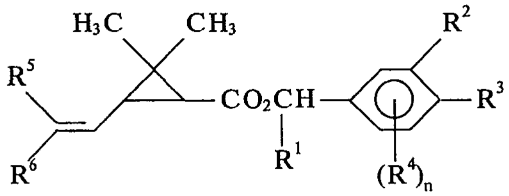

(XIII)

dans laquelle

$R^1$ est l'hydrogène ou le groupe cyano,
$R^2$ est le groupe trifluorométhoxy,
$R^3$ est l'hydrogène ou le fluor ou bien
$R^2$ et $R^3$ forment ensemble un groupe difluorodioxyméthylène,
$R^4$ est le fluor ou le chlore et
n peut avoir la valeur 0, 1, 2 ou 3, sous réserve que, au cas où $R^2$ représente un groupe $OCF_3$, l'un des restes $R^3$ ou $R^4$ doive représenter du fluor et sous réserve en outre que lorsque $R^2$ et $R^3$ représentent un groupe dioxyméthylène substitué par du fluor, n soit égal à 1 et $R^4$ doive représenter du fluor en position 6,
$R^5$ et $R^6$ sont égaux ou différents et représentent un groupe méthyle, un halogène ou un groupe halogénalkyle ayant 1 à 4 atomes de carbone.

8. Esters benzyliques suivant la revendication 7, de formule (XIII) dans laquelle
$R^1$–$R^4$ et n ont les définitions données dans la revendication 7,

$R^5$ représente le chlore et
$R^6$ représente du chlore, un groupe trifluorométhyle ou un groupe pentafluoréthyle.

9. Compositions pesticides, caractérisées par une teneur en au moins un ester benzylique de formule (I).

10. Utilisation des esters benzyliques de formule (I) pour combattre des parasites.